# EUROPEAN PATENT APPLICATION

(11) **EP 2 058 000 A1**
(43) Date of publication of application: **13.05.2009**
(21) Application number: 07120289.9
(22) Date of filing: 08.11.2007
(51) Int. Cl.: A61K 39/12

(54) **Immunogenic compositions capable of activating T cells**

(71) Applicant: Crossbeta Biosciences B.V., 3584 CX Utrecht (NL)
(72) Inventor: The designation of the inventor has not yet been filed
(74) Representative: Hatzmann, Martin

(57) **Abstract**

The invention provides means and methods for producing and/or selecting immunogenic compositions capable of activating a T-cell and/or a T-cell response, comprising providing said composition with at least one crossbeta structure and testing at least one immunogenic property.

## Description

The invention relates to the fields of cell biology, immunology, vaccinology, adjuvant technology and medicine.

Vaccines can be divided in two basic groups, i.e. prophylactic vaccines and therapeutic vaccines. Prophylactic vaccines have been made and/or suggested against essentially every known infectious agent (virus, bacterium, yeast, fungi, parasite, mycoplasm, etc.), which has some pathology in man, pets and/or livestock, which infectious agent is therefore also referred to as *pathogen.* Therapeutic vaccines have been made and/or suggested for infectious agents as well, but also for treatments of cancer and other aberrancies, as well as for inducing immune responses against other self antigens, as widely ranging as e.g. LHRH for immunocastration of boars, or for use in preventing graft versus host (GvH) and/or transplant rejections.

In vaccines in general there are two vital issues. Vaccines have to be efficacious and vaccines have to be safe. It often seems that the two requirements are mutually exclusive when trying to develop a vaccine. The most efficacious vaccines so far have been modified live infectious agents. These are modified in a manner that their virulence has been reduced (attenuation) to an acceptable level. The vaccine strain of the infectious agent typically does replicate in the host, but at a reduced level, so that the host can mount an adequate immune response, also providing the host with long term immunity against the infectious agent. The downside of attenuated vaccines is that the infectious agents may revert to a more virulent (and thus pathogenic) form.

This may happen in any infectious agent, but is a very serious problem in fast mutating viruses (such as in particular RNA viruses). Another problem with modified live vaccines is that infectious agents often have many different serotypes. It has proven to be difficult in many cases to provide vaccines which elicit an immune response in a host that protects against different serotypes of infectious agents.

Vaccines in which the infectious agent has been killed are often safe, but often their efficacy is mediocre at best, even when the vaccine contains an adjuvant. In general an immune response is enhanced by adding adjuvants (from the Latin *adjuνare,* meaning "to help") to the vaccines. The chemical nature of adjuvants, their proposed mode of action and their reactions (side effect) are highly variable. Some of the side effects can be ascribed to an unintentional stimulation of different mechanisms of the immune system whereas others may reflect general adverse pharmacological reactions which are more or less expected. There are several types of adjuvants. Today the most common adjuvants for human use are aluminium hydroxide, aluminium phosphate and calcium phosphate. However, there is a number of other adjuvants based on oil emulsions, products from bacteria (their synthetic derivatives as well as liposomes) or gram-negative bacteria, endotoxins, cholesterol, fatty acids, aliphatic amines, paraffinic and vegetable oils. Recently, monophosphoryl lipid A, ISCOMs with Quil-A, and Syntex adjuvant formulations (SAFs) containing the threonyl derivative or muramyl dipeptide have been under consideration for use in human vaccines. Chemically, adjuvants are a highly heterogenous group of compounds with only one thing in common: their ability to enhance the immune response-their adjuvanticity. They are highly variable in terms of how they affect the immune system and how serious their adverse effects are due to the resultant hyperactivation of the immune system. The choice of any of these adjuvants reflects a compromise between a requirement for adjuvanticity and an acceptable low level of adverse reactions. The term *adjuvant* has been used for any material that is capable of increasing the humoral and/or cellular immune response to an antigen. In the conventional vaccines, adjuvants are used to elicit an early, high and long-lasting immune response. The newly developed purified subunit or synthetic vaccines (see below) using biosynthetic, recombinant and other modern technology are poor immunogens and require adjuvants to evoke the immune response. The use of adjuvants enables the use of less antigen to achieve the desired immune response, and this reduces vaccine production costs. With a few exceptions, adjuvants are foreign to the body and cause adverse reactions.

A type of vaccine that has received a lot of attention since the advent of modern biology is the subunit vaccine. In these vaccines only one or a few elements of the infectious agent are used to elicit an immune response. Typically a subunit vaccine comprises one, two or three proteins, glycoproteins and/or peptides present in proteins, or fragments thereof, of an infectious agent (from one or more serotypes) which have been purified from a pathogen or produced by recombinant means and/or synthetic means. Although these vaccines in theory are the most promising safe and efficacious vaccines, in practice efficacy has proved to be a major hurdle. Molecular biology has provided more alternative methods to arrive at safe and efficacious vaccines that theoretically should also provide cross-protection against different serotypes of infectious agents. Carbohydrate structures derived from infectious agents have been suggested as specific immune response eliciting components of vaccines, as well as lipopolysaccharide structures, and even nucleic acid complexes have been proposed. Although these component vaccines are generally safe, their efficacy and cross-protection over different serotypes has been generally lacking. Combinations of different kinds of components have been suggested (carbohydrates with peptides/proteins and lipopolysaccharide (LPS) with peptides/proteins optionally with carriers), but so far the safety vs. efficacy issue remains.

Another approach to provide cross protection is to make hybrid infectious agents which comprise antigenic components from two or more serotypes of an infectious agent. These can be and have been produced by modern molecular biology techniques. They can be produced as modified live vaccines, or as vaccines with inactivated or killed pathogens, but also as subunit vaccines. Cocktail or combination vaccines comprising antigens from completely different infectious agents are also well known. In many countries children are routinely vaccinated with cocktail vaccines against e.g. diphteria, whooping cough, tetanus and polio. Recombinant vaccines comprising antigenic elements from different infectious agents have also been suggested. For instance for poultry a vaccine based on a chicken anemia virus has been suggested to be complemented with antigenic elements of Marek disease virus (MDV), but many more combinations have been suggested and produced.

Another important advantage of modern recombinant vaccines is that they have provided the opportunity to produce marker vaccines. Marker vaccines have been provided with an extra element that is not present in wild type infectious agent, or marker vaccines lack an element that is present in wild type infectious agent. The response of a host to both types of marker vaccines can be distinguished (typically by serological diagnosis) from the response against an infection with wild type.

An efficient way of producing immunogenic compositions, or improving the immunogenicity of immunogenic compositions, has been provided in WO 2007/008070. This patent application discloses that the immunogenicity of a composition which comprises amino acid sequences is enhanced by providing said composition with at least one crossbeta structure. A crossbeta structure is a structural element of peptides and proteins, comprising stacked beta sheets, as will be discussed in more detail below. According to WO 2007/008070, the presence of crossbeta structure enhances the immunogenicity of a composition comprising an amino acid sequence. An immunogenic composition is thus prepared by producing a composition which comprises an amino acid sequence, such as a protein containing composition, and administrating (protein comprising) crossbeta structures to said composition. Additionally, or alternatively, crossbeta structure formation in said composition is induced, for instance by changing the pH, salt concentration, reducing agent concentration, temperature, buffer and/or chaotropic agent concentration, and/or combinations of these parameters.

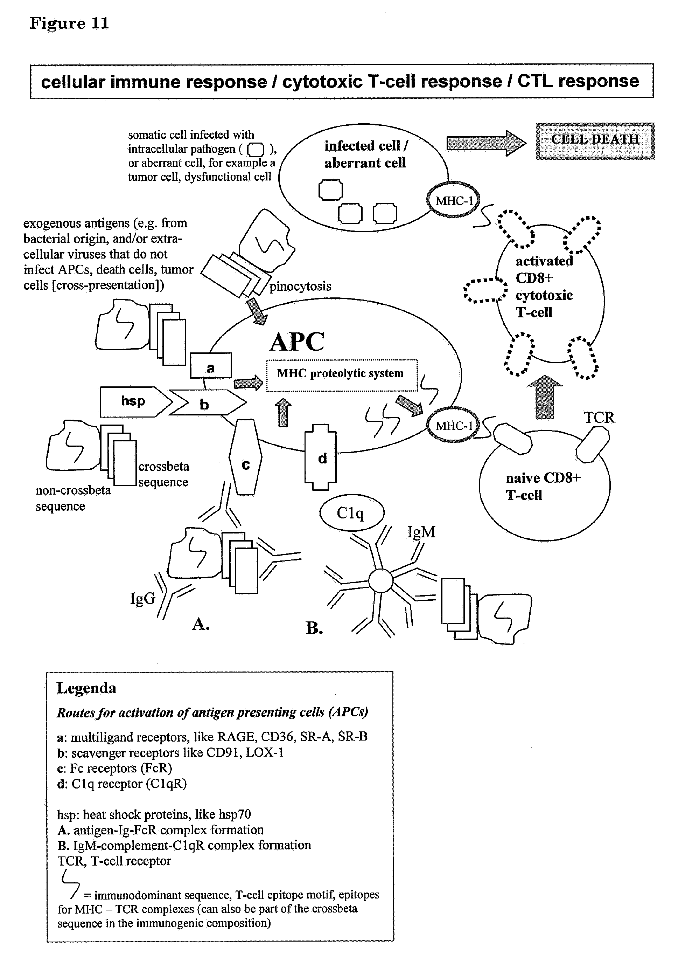

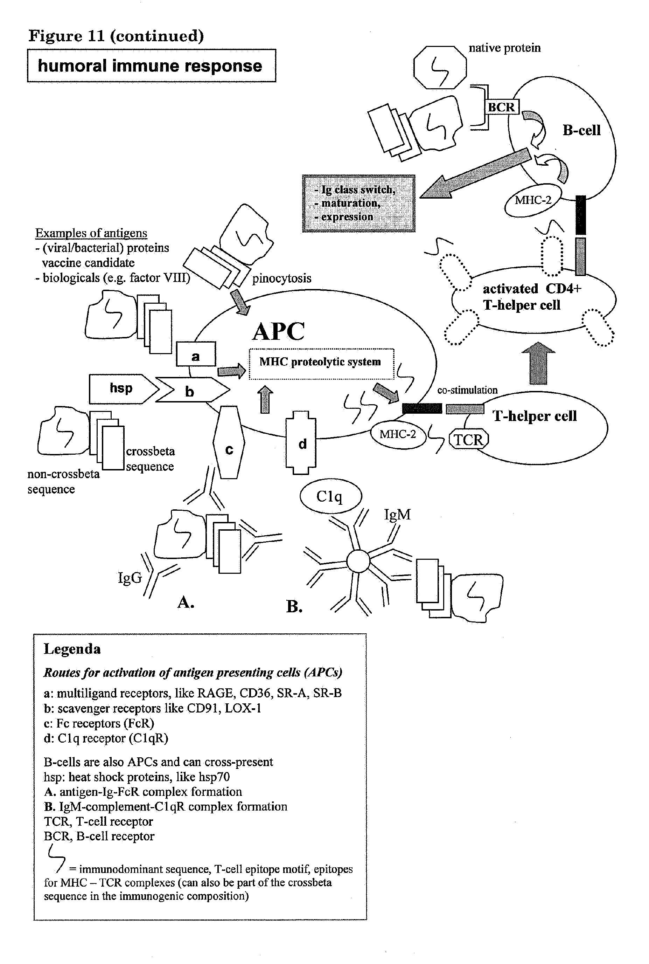

The methods disclosed in WO 2007/008070 are suitable for the production of immunogenic compositions capable of eliciting and/or stimulating a humoral immune response, as well as for the production of immunogenic compositions capable of eliciting and/or stimulating a cellular immune response. A schematic overview of a humoral and a cellular immune response is given in Figure 11. A humoral immune response, including the production of antigen-specific antibodies, is often elicited against extracellular pathogens such as virus, bacteria, yeast, fungi, parasite and mycoplasm whereas a cellular immune response, including the production of a cytotoxic T-cell response, is often elicited against intracellular pathogens, cancer and self-antigens.

It is an object of the present invention to provide improved means and methods for producing and/or improving immunogenic compositions. It is a further object to provide compositions with enhanced immunogenicity for use as vaccines.

In one preferred embodiment the present invention provides improved methods for providing and/or selecting immunogenic compositions which are capable of activating T-cells, for example resulting in a CD4+ T-help response, and/or resulting in a CD8+ cytotoxic T-lymphocyte response. T-cell epitopes are not always known. When T-cell epitope motifs are not known for a given protein, T-cell epitope motifs are preferably predicted. It is possible to predict T-cell epitopes for CD4+ related T-cell activation as well as for T-cell epitopes for CD8+ related T-cell activation. It is known in the art how T-cell epitopes capable of inducing an MHC-I mediated T-cell activation as well as T-cell epitopes capable of inducing an MHC-II mediated T-cell activation are predicted. This is for instance done by screening the primary sequence of a compound comprising an amino acid sequence such as, but not limited to, a peptide, polypeptide, protein, glycoprotein, protein-DNA complex, protein-membrane complex and/or lipoprotein, and in summary referred to as 'protein', for the presence of peptides with a length of between 5-30 amino-acid residues, preferably flanked by sequences which are capable of being recognized and cleaved by an MHC antigen processing pathway. It is preferably also determined whether putative T-cell epitopes have anchor residues so that the epitopes can be bound to a component of an MHC antigen processing pathway and be presented by an antigen presenting cell. Putative T-cell epitope motifs are for example obtained by synthesizing peptides covering overlapping sequences of the antigen, comprising preferably the number of amino-acid residues known to be required for presentation by major histocompatibility complexes, for example 5-30 amino-acid residues. The sequence overlap between two adjacent peptides is for example 1-10 amino-acid residues. Algorithms and computer based analysis techniques are often used in order to determine whether a protein comprises T-cell epitope motifs.

According to the present invention, at least one peptide, polypeptide, protein, glycoprotein, protein-DNA complex, protein-membrane complex and/or lipoprotein comprising at least one predicted and/or putative T-cell epitope motif is selected and incorporated into one or more compositions. Subsequently, said composition is provided with at least one crossbeta structure. This way, an immunogenic composition capable of eliciting and/or stimulating a cellular immune response is obtained.

One embodiment of the present invention thus provides a method for producing an immunogenic composition comprising at least one peptide, polypeptide, protein, glycoprotein, protein-DNA complex, protein-membrane complex and/or lipoprotein, the method comprising:
- determining whether a peptide, polypeptide, protein, glycoprotein, protein-DNA complex, protein-membrane complex and/or lipoprotein comprises a T-cell epitope motif;
- selecting a peptide, polypeptide, protein, glycoprotein, protein-DNA complex, protein-membrane complex and/or lipoprotein comprising a T-cell epitope motif;
- providing a composition comprising said selected peptide, polypeptide, protein, glycoprotein, protein-DNA complex, protein-membrane complex and/or lipoprotein; and
- providing said composition with at least one crossbeta structure.

One advantage of the use of a crossbeta structure is that the use of adjuvants in order to induce an immune response is reduced or no longer necessary (although such adjuvant may still be used at will).

It is of course also possible to use a peptide, polypeptide, protein, glycoprotein, protein-DNA complex, protein-membrane complex and/or lipoprotein which is known to comprise a T-cell epitope. A composition comprising such T-cell epitope comprising compound is provided with crossbeta structures in order to obtain an immunogenic compound.

In one embodiment, said T-cell epitope comprises a cytotoxic T lymphocyte (CTL) epitope. This way an immunogenic composition capable of eliciting and/or stimulating a cellular immune response is obtained.

Alternatively, or additionally, said T-cell epitope comprises a T-helper cell epitope. In this embodiment an immunogenic composition capable of eliciting and/or stimulating a humoral immune response is obtained.

The present invention furthermore provides improved methods for providing an immunogenic composition capable of activating T-cells and/or a T-cell response, the method comprising providing an amino acid containing composition with at least one crossbeta structure and subsequently testing at least one, preferably at least two, immunogenic properties of the resulting composition. The present invention thus provides a way for controlling a process for the production of an immunogenic composition, so that immunogenic compositions with preferred immunogenic properties are produced and/or selected. In this embodiment a peptide, polypeptide, protein, glycoprotein, protein-DNA complex, protein-membrane complex and/or lipoprotein comprising a known T-cell epitope and/or a predicted or determined T-cell epitope motif is used. The present invention provides a method wherein a composition comprising at least one amino acid sequence such as, but not limited to, a peptide, polypeptide, protein, glycoprotein, protein-DNA complex, protein-membrane complex and/or lipoprotein, which comprises a T-cell epitope and/or a T-cell epitope motif, is provided with at least one crossbeta structure, where after at least one of the following properties is tested:
- whether the degree of multimerization of said peptide, polypeptide, protein, glycoprotein, protein-DNA complex, protein-membrane complex and/or lipoprotein in said composition allows recognition, excision, processing and/or presentation of a T-cell epitope of said peptide, polypeptide, protein, glycoprotein, protein-DNA complex, protein-membrane complex and/or lipoprotein by an animal's immune system;
- whether between 4-75% of the peptide, polypeptide, protein, glycoprotein, protein-DNA complex, protein-membrane complex and/or lipoprotein content of said composition is in a conformation comprising crossbeta structures;
- whether said at least one crossbeta structure comprises a property allowing recognition, excision, processing and/or presentation of a T-cell epitope of said peptide, polypeptide, protein, glycoprotein and/or lipoprotein by an animal's immune system; and/or
- whether a compound capable of specifically binding, recognizing, excising, processing and/or presenting a T-cell epitope of said peptide, polypeptide, protein, glycoprotein, protein-DNA complex, protein-membrane complex and/or lipoprotein is capable of specifically binding, recognizing, excising, processing and/or presenting said immunogenic composition.

This is outlined below in more detail.

Crossbeta structures are present in a subset of misfolded proteins such as for instance amyloid. A misfolded protein is defined herein as a protein with a structure other than a native, non-amyloid, non-crossbeta structure. Hence, a misfolded protein is a protein having a non-native three dimensional structure, and/or a crossbeta structure, and/or an amyloid structure.

Misfolded proteins tend to multimerize and can initiate fibrillization. This can result in the formation of amorphous aggregates that can vary greatly in size. In certain cases misfolded proteins are more regular and fibrillar in nature. The term *amyloid* has initially been introduced to define the fibrils, which are formed from misfolded proteins, and which are found in organs and tissues of patients with the various known misfolding diseases, collectively termed amyloidoses. Commonly, amyloid appears as fibrils with undefined length and with a mean diameter of 10 nm, is deposited extracellularly, stains with the dyes Congo red and Thioflavin T (ThT), shows characteristic green birefringence under polarized light when Congo red is bound, comprises beta-sheet secondary structure, and contains the characteristic crossbeta conformation (see below) as determined by X-ray fibre diffraction analysis. However, since it has been determined that protein misfolding is a more general phenomenon and since many characteristics of misfolded proteins are shared with amyloid, the term *amyloid* has been used in a broader scope. Now, the term *amyloid* is also used to define intracellular fibrils and fibrils formed in vitro. Also the terms *amyloid-like* and *amylog* are used to indicate misfolded proteins with properties shared with amyloids, but that do not fulfil all criteria for amyloid, as listed above.

In conclusion, misfolded proteins are highly heterogeneous in nature, ranging from monomeric misfolded proteins, to small oligomeric species, sometimes referred to as protofibrils, larger aggregates with amorphous appearance, up to large highly ordered fibrils, all of which appearances can share structural features reminiscent to amyloid. As used herein, the term "misfoldome" encompasses any collection of misfolded proteins.

Amyloid and misfolded proteins that do not fulfil all criteria for being identified as amyloid can share structural and functional features with amyloid and/or with other misfolded proteins. These common features are shared among various misfolded proteins, independent of their varying amino acid sequences. Shared structural features include for example the binding to certain dyes, such as Congo red, ThT, Thioflavin S, accompanied by enhanced fluorescence of the dyes, multimerization, and the binding to certain proteins, such as tissue-type plasminogen activator (tPA), the receptor for advanced glycation end-products (RAGE) and chaperones, such as heat shock proteins, like BiP (grp78 or immunoglobulin heavy chain binding protein). Shared functional activities include the activation of tPA and the induction of cellular responses, such as inflammatory responses and an immune response, and induction of cell toxicity.

A unique hallmark of a subset of misfolded proteins such as for instance amyloid is the presence of the crossbeta conformation or a precursor form of the crossbeta conformation.

A crossbeta structure is a secondary structural element in peptides and proteins. A crossbeta structure (also referred to as a "cross-β", a "cross beta" or a "cross-β structure") is defined as a part of a protein or peptide, or a part of an assembly of peptides and/or proteins, which comprises single beta-strands (stage 1) and a(n ordered) group of beta-strands (stage 2), and typically a group of beta-strands, preferably composed of 5-10 beta-strands, arranged in a beta-sheet (stage 3). A crossbeta structure often comprises in particular a group of stacked beta-sheets (stage 4), also referred to as "amyloid". A crossbeta structure is formed following formation of a crossbeta structure precursor form upon protein misfolding like for example denaturation, proteolysis or unfolding of proteins. A crossbeta structure precursor is defined as any protein conformation that precedes the formation of any of the aforementioned structural stages of a crossbeta structure. These structural elements present in crossbeta structure (precursor) are typically absent in globular regions of (native parts of) proteins. The presence of crossbeta structure is for example demonstrated with X-ray fibre diffraction or binding of ThT or binding of Congo red, accompanied by enhanced fluorescence of the dyes.

A typical form of a crossbeta structure precursor is a partially or completely misfolded protein. A typical form of a misfolded protein is a partially or completely unfolded protein, a partially refolded protein, a partially or completely aggregated protein, an oligomerized or multimerized protein, or a partially or completely denatured protein. A crossbeta structure or a crossbeta structure precursor can appear as monomeric molecules, dimeric, trimeric, up till oligomeric assemblies of molecules and can appear as multimeric structures and/or assemblies of molecules.

Crossbeta structure (precursor) in any of the aforementioned states can appear in soluble form in aqueous solutions and/or organic solvents and/or any other solutions. Crossbeta structure (precursor) can also be present as solid state material in solutions, like for example as insoluble aggregates, fibrils, particles, like for example as a suspension or separated in a solid crossbeta structure phase and a solvent phase.

Protein misfolding, formation of crossbeta structure precursor, formation of aggregates or multimers and/or crossbeta structure can occur in any composition comprising protein(s) and/or peptides with a length of at least 2 amino acids. The term "peptide" is intended to include oligopeptides as well as polypeptides, and the term "protein" includes proteinaceous molecules including peptides, with and without post-translational modifications such as for instance glycosylation, citrullination, oxidation, acetylation and glycation. It also includes lipoproteins and complexes comprising a proteinaceous part, such as for instance protein-nucleic acid complexes (RNA and/or DNA), membrane-protein complexes, etc. As used herein, the term "protein" also encompasses proteinaceous molecules, peptides, oligopeptides and polypeptides. Hence, the use of "protein" or "protein and/or peptide" in this application have the same meaning.

A typical form of stacked beta-sheets is in a fibril-like structure in which the beta-sheets are stacked in either the direction of the axis of the fibril or perpendicular to the direction of the axis of the fibril. The direction of the stacking of the beta-sheets in crossbeta structures is perpendicular to the long fiber axis. A crossbeta structure conformation is a signal that triggers a cascade of events that induces clearance and breakdown of the obsolete protein or peptide. When clearance is inadequate, unwanted proteins and/or peptides aggregate and form toxic structures ranging from soluble oligomers up to precipitating fibrils and amorphous plaques. Such crossbeta structure conformation comprising aggregates underlie various diseases and disorders, such as for instance, Huntington's disease, amyloidosis type disease, atherosclerosis, cardiovascular disease, diabetes, bleeding, thrombosis, cancer, sepsis and other inflammatory diseases, rheumatoid arthritis, transmissible spongiform encephalopathies such as Creutzfeldt-Jakob disease, multiple sclerosis, auto-immune diseases, uveitis, ankylosing spondylitis, diseases associated with loss of memory such as Alzheimer's disease, Parkinson's disease and other neuronal diseases (epilepsy), encephalopathy and systemic amyloidoses.

A crossbeta structure is for instance formed during unfolding and refolding of proteins and peptides. Unfolding of peptides and proteins occur regularly within an organism. For instance, peptides and proteins often unfold and refold spontaneously at the end of their life cycle. Moreover, unfolding and/or refolding is induced by environmental factors such as for instance pH, glycation, oxidative stress, heat, irradiation, mechanical stress, proteolysis citrullination, ischeamia, and so on. As used herein, the terms *crossbeta* and *crossbeta structure* also encompasses any crossbeta structure precursor and any misfolded protein, even though a misfolded protein does not necessarily comprise a crossbeta structure. The term "crossbeta binding molecule" or "molecule capable of specifically binding a crossbeta structure" also encompasses a molecule capable of specifically binding any misfolded protein.

The terms unfolding, refolding and misfolding relate to the three-dimensional structure of a protein or peptide. Unfolding means that a protein or peptide loses at least part of its three-dimensional structure. The term refolding relates to the coiling back into some kind of three-dimensional structure. By refolding, a protein or peptide can regain its native configuration, or an incorrect refolding can occur. The term "incorrect refolding" refers to a situation when a three-dimensional structure other than a native configuration is formed. Incorrect refolding is also called misfolding. Unfolding and refolding of proteins and peptides involves the risk of crossbeta structure formation. Formation of crossbeta structures sometimes also occurs directly after protein synthesis, without a correctly folded protein intermediate.

In a method according to the present invention, an immunogenic composition comprising at least one peptide, polypeptide, protein, glycoprotein, protein-DNA complex, protein-membrane complex and/or lipoprotein is provided with at least one crossbeta structure. This is performed in various ways. For instance, a peptide, polypeptide, protein, glycoprotein, protein-DNA complex, protein-membrane complex and/or lipoprotein is subjected to a crossbeta inducing procedure, preferably a change of pH, salt concentration, reducing agent concentration, temperature, buffer and/or chaotropic agent concentration. These procedures are known to induce and/or enhance crossbeta formation. In one embodiment said peptide, polypeptide, protein, glycoprotein, protein-DNA complex, protein-membrane complex and/or lipoprotein is subjected to a crossbeta inducing procedure before it is used for the preparation of an immunogenic composition. It is, however, also possible to subject said peptide, polypeptide, protein, glycoprotein, protein-DNA complex, protein-membrane complex and/or lipoprotein to a crossbeta inducing procedure while it is already present in an immunogenic composition.

Additionally, or alternatively, a peptide, polypeptide, protein, glycoprotein, protein-DNA complex, protein-membrane complex and/or lipoprotein is provided with a (peptide or protein comprising a) crossbeta structure, either before it is used for the preparation of an immunogenic composition or after it has been used for the preparation of an immunogenic composition.

After an immunogenic composition according to the invention has been provided with crossbeta structures, one or more immunogenic properties of the resulting composition are tested.

In one embodiment it is tested whether the degree of multimerization of the peptide, polypeptide, protein, glycoprotein, protein-DNA complex, protein-membrane complex and/or lipoprotein in said immunogenic composition allows recognition, excision, processing and/or presentation of a T-cell epitope of said peptide, polypeptide, protein, glycoprotein, protein-DNA complex, protein-membrane complex and/or lipoprotein by an animal's immune system. Proteins comprising crossbeta structures tend to multimerize. Hence, after an immunogenic composition has been provided with crossbeta structures, multimerization of the peptide, polypeptide, protein, glycoprotein, protein-DNA complex, protein-membrane complex and/or lipoprotein in said immunogenic composition will occur. According to the present invention, it is tested whether the degree of multimerization is such that an animal's immune system is still capable of recognizing, excising, processing and/or presenting a T-cell epitope (of interest). For instance, too much multimerization will result in the formation of a fibril wherein T-cell epitopes are no longer accessible for protease systems, for example the MHC antigen processing pathway. Additionally, or alternatively, too much multimerization results in a decreased ability of the crossbeta structures present in said peptide, polypeptide, protein, glycoprotein, protein-DNA complex, protein-membrane complex and/or lipoprotein of binding multiligand receptors and activating an animal's immune system.

Preferably monomers and/or multimers of the peptide, polypeptide, protein, glycoprotein, protein-DNA complex, protein-membrane complex and/or lipoprotein in said immunogenic composition have dimensions in the range of 0.5 nm to 1000 µm, and more preferably, in the range of 0.5 nm to 100 µm, and even more preferably in the range of 1 nm to 5 µm, and even more preferably in the range of 3 - 2000 nm. Obviously, this range of dimensions is determined by the number of protein molecules per multimer, with a given number of amino-acid residues per protein molecule. Therefore, the dimensions are alternatively and/or additively expressed in terms of number of protein monomers per multimer.

In another embodiment it is tested whether between 4-75% of the peptide, polypeptide, protein, glycoprotein, protein-DNA complex, protein-membrane complex and/or lipoprotein content of said composition is in a conformation comprising crossbeta structures. According to the invention, even though crossbeta structure enhances immunogenicity, the presence of too many crossbeta structures negatively influences immunogenicity. A crossbeta content between (and including) 4 and 75% is preferred. It is possible to determine the ratio between total crossbeta structure and total protein content. In a preferred embodiment, however, the crossbeta content within single proteins is determined. Preferably, individual proteins have a crossbeta content of between (and including) 4 and 75%, so that at least one epitope remains available for an animal's immune system. Most preferably, at least 70% of the individual proteins each have a crossbeta content of between (and including) 4 and 75%.

In another embodiment it is tested whether said at least one crossbeta structure comprises a property allowing recognition, excision, processing and/or presentation of a T-cell epitope of said peptide, polypeptide, protein, glycoprotein, protein-DNA complex, protein-membrane complex and/or lipoprotein by an animal's immune system. Recognition of a crossbeta structure by a component of an animal's immune system, for instance by a multiligand receptor, such as but not limited to LRP, CD36, RAGE, SR-A, or LOX-1, results in (the initiation of) an immunogenic reaction against a peptide, polypeptide, protein, glycoprotein, protein-DNA complex, protein-membrane complex and/or lipoprotein of an immunogenic composition according to the invention (see for instance Figure 11). It is therefore preferably tested whether a crossbeta structure of an immunogenic composition according to the invention has a desired (binding) property.

In another embodiment it is tested whether a compound capable of specifically binding, recognizing, excising, processing and/or presenting a T-cell epitope of said peptide, polypeptide, protein, glycoprotein, protein-DNA complex, protein-membrane complex and/or lipoprotein is capable of specifically binding, recognizing, excising, processing and/or presenting said T-cell epitope. In principle, induction and/or administration of a crossbeta structure into a composition could result in a diminished availability of a T-cell epitope of interest. For instance, if a crossbeta structure is induced in a region of a peptide or protein wherein an epitope is present, said epitope is at risk of being shielded. The conformation of said epitope is also at risk of being disturbed. Alternatively, if a peptide sequence of a composition is coupled to a crossbeta containing peptide or protein, the coupling could take place at the site of an epitope of interest, thereby reducing its availability for an animal's immune system. In short, the availability of a T-cell epitope of interest for an animal's immune system could be diminished after an immunogenic composition has been provided with crossbeta structures. This is in one embodiment tested by determining whether a compound which is capable of specifically binding, recognizing, excising, processing and/or presenting a T-cell epitope of interest is still capable of binding, recognizing, excising, processing and/or presenting said T-cell epitope after the composition has been provided with crossbeta structure. If said compound is capable of specifically binding, recognizing, excising, processing and/or presenting said T-cell epitope, it shows that said epitope is still available for an animal's immune system. Said compound for instance comprises an intracellular protease capable of excising said T-cell epitope from the primary amino acid sequence of an antigen. In one preferred embodiment said compound comprises a component of a MHC complex. Said MHC complex comprises either MHC-I and/or MHC-II. In another embodiment, said compound comprises a T-cell or a T-cell receptor. The ability of an immunogenic composition comprising amino-acid sequences with crossbeta conformation, referred to as 'crossbeta-antigens', to induce (primary) T cell responses in *vivo* is preferably tested *in vitro* using T-cells isolated from immunized animals, for example mammals. For example, T cells are isolated from mice. In one embodiment, T-cells from a human individual who has been exposed to an antigen such as a pathogen are used. Alternatively, activation of naïve T cells is analyzed upon isolation of T-cells from non-immunized animals, for example mammals, for example from mice or human individuals.

Several methods for T-cell isolation are known and commonly used in practice by persons skilled in the art. Preferably, T-cells are isolated from blood or splenocytes, for example from splenocytes isolated from immunized mammals, for example mice. In one embodiment non-human mammals, for example mice are immunized with antigen, preferably immunogenic compositions comprising crossbeta adjuvant and peptide, polypeptide, protein, glycoprotein, protein-DNA complex, protein-membrane complex and/or lipoprotein comprising at least one T-cell epitope and/or T-cell epitope motif, preferably once or twice, and cells are isolated preferably between 3 and 14 days after immunization. Preferably, spleen cell suspensions or peripheral blood mononuclear cells are used. Splenocytes are preferably isolated using cell strainers, preferably with a pore size of 100 µm. Preferably, erythrocytes are removed from the cell suspension, preferably by a centrifugation step using Ficoll, or by hemolysis, preferably with a hypotonic buffer, preferably composed of ammonium chloride, preferably at 0.15 mM, and potassium bicarbonate, preferably at 0.1 mM, and ethylendiaminetetaacetic acid, preferably at 0.01 mM.

Subsequently, isolated and washed T-cells are used for analysis of their response towards immunogenic compositions comprising crossbeta adjuvant and peptide, polypeptide, protein, glycoprotein, protein-DNA complex, protein-membrane complex and/or lipoprotein comprising at least one T-cell epitope motif. For example, such analyses are performed in an indirect way with antigen presenting cells included in the analysed cell cultures, and/or directly by assessing responsiveness towards T-cell epitope motifs, for example using peptides of such motifs.

If said immunogenic composition appears to be capable of eliciting and/or stimulating a T-cell response, it shows that at least one T-cell epitope is still available for an animal's immune system.

In a preferred embodiment, at least two of the above mentioned tests are carried out. Of course, any combination of tests is possible. In one embodiment at least three of the above mentioned tests are carried out.

The present invention thus provides a method for producing an immunogenic composition which is capable of activating T-cells and/or a T-cell response, the composition comprising at least one peptide, polypeptide, protein, glycoprotein, protein-DNA complex, protein-membrane complex and/or lipoprotein comprising a T-cell epitope and/or a T-cell epitope motif, the method comprising providing said composition with at least one crossbeta structure and determining:
- whether the degree of multimerization of said peptide, polypeptide, protein, glycoprotein, protein-DNA complex, protein-membrane complex and/or lipoprotein in said composition allows recognition, excision, processing and/or presentation of a T-cell epitope of said peptide, polypeptide, protein, glycoprotein, protein-DNA complex, protein-membrane complex and/or lipoprotein by an animal's immune system;
- whether between 4-75% of the peptide, polypeptide, protein, glycoprotein, protein-DNA complex, protein-membrane complex and/or lipoprotein content of said composition is in a conformation comprising crossbeta structures;
- whether said at least one crossbeta structure comprises a property allowing recognition, excision, processing and/or presentation of a T-cell epitope of said peptide, polypeptide, protein, glycoprotein and/or lipoprotein by an animal's immune system; and/or
- whether a compound capable of specifically binding, recognizing, excising, processing and/or presenting a T-cell epitope of said peptide, polypeptide, protein, glycoprotein, protein-DNA complex, protein-membrane complex and/or lipoprotein is capable of specifically binding, recognizing, excising, processing and/or presenting a T-cell epitope of said peptide.

In one preferred embodiment it is determined whether monomers and/or multimer of the peptide, polypeptide, protein, glycoprotein, protein-DNA complex, protein-membrane complex and/or lipoprotein in said immunogenic composition have dimensions in the range of 0.5 nm to 1000 µm, and more preferably, in the range of 0.5 nm to 100 µm, and even more preferably in the range of 1 nm to 5 µm, and even more preferably in the range of 3 - 2000 nm. Obviously, this range of dimensions is determined by the number of protein molecules per multimer, with a given number of amino-acid residues per protein molecule. Therefore, the dimensions are alternatively and/or additively expressed in terms of number of protein monomers per multimer.

An animal comprises any animal having an immune system, preferably a mammal. In one preferred embodiment said animal comprises a human individual.

A protein-membrane complex is defined as a compound or composition comprising an amino acid sequence as well as a lipid molecule, and/or a fragment thereof, and/or a derivative thereof, for example assemblied in a membrane and/or vesicle and/or liposome type of arrangement.

*An immunogenic composition comprising at least one peptide, polypeptide, protein, glycoprotein, protein-DNA complex, protein-membrane complex and*/*or lipoprotein* is defined herein as a composition comprising at least one amino acid sequence, which composition is capable of eliciting and/or enhancing an immune response in an animal, preferably a mammal, against at least part of said peptide, polypeptide, protein, glycoprotein, protein-DNA complex, protein-membrane complex and/or lipoprotein after administration of said immunogenic composition to said animal. Said immune response preferably comprises a humoral immune response and/or a cellular immune response. Said immune response need not be protective, therapeutic and/or capable of diminishing a consequence of disease. An immunogenic composition according to the invention is preferably capable of inducing and/or enhancing the formation of antibodies, and/or activating B-cells and/or T-cells which are capable of specifically binding an epitope of said peptide, polypeptide, protein, glycoprotein, protein-DNA complex, protein-membrane complex and/or lipoprotein.

In one preferred embodiment it is determined whether a proteolytic system, for example the MHC antigen processing pathway, is capable of binding, recognizing, excising, processing and/or presenting a T-cell epitope of said peptide, polypeptide, protein, glycoprotein, protein-DNA complex, protein-membrane complex and/or lipoprotein in the context of either MHC-I and/or MHC-II.

In another preferred embodiment it is determined whether said immunogenic composition and/or crossbeta structure is capable of specifically binding a crossbeta structure binding compound, preferably at least one compound selected from the group consisting of tPA, BiP, factor XII, fibronectin, hepatocyte growth factor activator, at least one finger domain of tPA, at least one finger domain of factor XII, at least one finger domain of fibronectin, at least one finger domain of hepatocyte growth factor activator, Thioflavin T, Thioflavin S, Congo Red, CD14, a multiligand receptor such as RAGE or CD36 or CD40 or LOX-1 or TLR2 or TLR4, a crossbeta-specific antibody, preferably crossbeta-specific IgG and/or crossbeta-specific IgM, IgIV, an enriched fraction of IgIV capable of specifically binding a crossbeta structure, Low density lipoprotein Related Protein (LRP), LRP Cluster II, LRP Cluster IV, Scavenger Receptor B-1 (SR-BI), SR-A, chrysamine G, a chaperone, a heat shock protein, HSP70, HSP60, HSP90, gp95, calreticulin, a chaperonin, a chaperokine and a stress protein.

If said immunogenic composition appears to be capable of specifically binding such crossbeta binding compound, it shows that said immunogenic composition comprises a crossbeta structure which is capable of inducing and/or activating an animal's immune system.

Molecular chaperones are a diverse class of proteins comprising heat shock proteins, chaperonins, chaperokines and stress proteins, that are contributing to one of the most important cell defence mechanisms that facilitates protein folding, refolding of partially denatured proteins, protein transport across membranes, cytoskeletal organization, degradation of disabled proteins, and apoptosis, but also act as cytoprotective factors against deleterious environmental stresses. Individual members of the family of these specialized proteins bind non-native states of one or several or whole series or classes of proteins and assist them in reaching a correctly folded and functional conformation. Alternatively, when the native fold cannot be achieved, molecular chaperones contribute to the effective removal of misfolded proteins by directing them to the suitable proteolytic degradation pathways. Chaperones selectively bind to non-natively folded proteins in a stable non-covalent manner. To direct correct folding of a protein from a misfolded form to the required native conformation, mostly several chaperones work together in consecutive steps.

Chaperonins are molecular machines that facilitate protein folding by undergoing energy (ATP)-dependent movements that are coordinated in time and space by complex allosteric regulation. Examples of chaperones that facilitate refolding of proteins from a misfolded conformation to a native form are heat shock protein (hsp) 90, hsp60 and hsp70. Chaperones also participate in the stabilization of unstable protein conformers and in the recovery of proteins from aggregates. Molecular chaperones are mostly heat- or stress-induced proteins (hsp's), that perform critical functions in maintaining cell homeostasis, or are transiently present and active in regular protein synthesis. Hsp's are among the most abundant intracellular proteins. Chaperones that act in an ATP-independent manner are for example the intracellular small hsp's, calreticulin, calnexin and extracellular clusterin. Under stress conditions such as elevated temperature, glucose deprivation and oxidation, small hsp's and clusterin efficiently prevent the aggregation of target proteins. Interestingly, both types of hsp's can hardly chaperone a misfolded protein to refold back to its native state. In patients with Creutzfeldt-Jakob, Alzheimer's disease and other diseases related to protein misfolding and accumulation of amyloid, increased expression of clusterin and small hsp's has been seen. Molecular chaperones are essential components of the quality control machineries present in cells. Due to the fact that they aid in the folding and maintenance of newly translated proteins, as well as in facilitating the degradation of misfolded and destabilized proteins, chaperones are essentially the cellular sensors of protein misfolding and function. Chaperones are therefore the gatekeepers in a first line of defence against deleterious effects of misfolded proteins, by assisting a protein in obtaining its native fold or by directing incorrectly folded proteins to a proteolytic breakdown pathway. Notably, hsp's are over-expressed in many human cancers. It has been established that hsp's play a role in tumor cell metastasis, proliferation, differentiation, invasion, death, and in triggering the immune system during cancer.

One of the key members of the quality control machinery of the cell is the ubiquitous molecular chaperone hsp90. Hsp90 typically functions as part of large complexes, which include other chaperones and essential cofactors that regulate its function. Different cofactors seem to target hsp90 to different sets of substrates. However, the mechanism of hsp90 function in protein misfolding biology remains poorly understood.

Intracellular pathways that are involved in sensing protein misfolding comprise the unfolded protein response machinery (UPR) in the endoplasmic reticulum (ER). Accumulation of unfolded and/or misfolded proteins in the ER induces ER stress resulting in triggering of the UPR. Environmental factors can transduce the stress response, like for example changes in pH, starvation, reactive oxygen species. During these episodes of cellular stress, intracellular heat shock proteins levels increase to provide cellular protection. Activation of the UPR includes the attenuation of general protein synthesis and the transcriptional activation of the genes encoding ER-resident chaperones and molecules involved in the ER-associated degradation (ERAD) pathway. The UPR reduces ER stress by restoration of the protein-folding capacity of the ER. A key protein acting as a sensor of protein misfolding is the chaperone BiP (also referred to as grp78; Immunoglobulin heavy chain-binding protein/ Endoplasmic reticulum luminal Ca²⁺-binding protein).

After testing of at least one immunogenic property of an immunogenic composition according to the present invention, an immunogenic composition with a desired property is preferably selected. If a desired property, such as the availability of a T-cell epitope of interest, appears not to be present (anymore) after a composition comprising at least one peptide, polypeptide, protein, glycoprotein, protein-DNA complex, protein-membrane complex and/or lipoprotein has been provided with crossbeta structures, another batch of the same kind of composition is preferably provided with crossbeta structures and tested again. If needed, this procedure is repeated until an immunogenic composition with at least one desired property/properties is obtained.

In one embodiment, an immunogenic composition is selected with a degree of multimerization of the peptide, polypeptide, protein, glycoprotein, protein-DNA complex, protein-membrane complex and/or lipoprotein which allows recognition, excision, processing and/or presentation of a T-cell epitope by an animal's immune system. Further provided is therefore a method according to the invention, further comprising selecting an immunogenic composition wherein the degree of multimerization of said peptide, polypeptide, protein, glycoprotein, protein-DNA complex, protein-membrane complex and/or lipoprotein in said composition allows recognition, excision, processing and/or presentation of a T-cell epitope of said peptide, polypeptide, protein, glycoprotein, protein-DNA complex, protein-membrane complex and/or lipoprotein by an animal's immune system.

In another embodiment, an immunogenic composition is selected with a crossbeta content of between 4-75% so that the immunogenicity is enhanced, while at least one epitope remains available for an animal's immune system. The term *immunogenicity* is defined herein as the capability of a compound or a composition to activate an animal's immune system. Of course, if it is intended that an animal's immune system is, at least in part, directed against an epitope of interest, said epitope of interest should be available for the animal's immune system. Further provided is therefore a method according to the invention, further comprising selecting an immunogenic composition wherein between 4-75% of the peptide, polypeptide, protein, glycoprotein, protein-DNA complex, protein-membrane complex and/or lipoprotein content of said composition is in a conformation comprising crossbeta structures.

In yet another embodiment an immunogenic composition is selected which comprises a crossbeta structure having a binding property which allows (the initiation of) an immunogenic reaction against a peptide, polypeptide, protein, glycoprotein, protein-DNA complex, protein-membrane complex and/or lipoprotein of an immunogenic composition according to the invention. Further provided is therefore a method according to the invention, further comprising selecting an immunogenic composition which comprises a crossbeta structure which is capable of specifically binding a crossbeta structure binding compound, preferably tPA, BiP, factor XII, fibronectin, hepatocyte growth factor activator, at least one finger domain of tPA, at least one finger domain of factor XII, at least one finger domain of fibronectin, at least one finger domain of hepatocyte growth factor activator, Thioflavin T, Thioflavin S, Congo Red, CD14, a multiligand receptor such as RAGE or CD36 or CD40 or LOX-1 or TLR2 or TLR4, a crossbeta-specific antibody, preferably crossbeta-specific IgG and/or crossbeta-specific IgM, IgIV, an enriched fraction of IgIV capable of specifically binding a crossbeta structure, Low density lipoprotein Related Protein (LRP), LRP Cluster II, LRP Cluster IV, Scavenger Receptor B-I (SR-BI), SR-A, chrysamine G, a chaperone, a heat shock protein, HSP70, HSP60, HSP90, gp95, calreticulin, a chaperonin, a chaperokine and/or a stress protein.

In yet another embodiment an immunogenic composition is selected whereby a proteolytic system, for example the MHC antigen processing pathway, is capable of recognizing, binding, excising, processing and/or presenting a T-cell epitope of said peptide, polypeptide, protein, glycoprotein, protein-DNA complex, protein-membrane complex and/or lipoprotein in the context of either MHC-I and/or MHC-II.

A method according to the invention is particularly suitable for selecting, from a plurality of immunogenic compositions, one or more immunogenic compositions having a greater chance of being capable of eliciting and/or stimulating a protective prophylactic immune response and/or a therapeutic immune response *in vivo*, as compared to the other immunogenic compositions of said plurality of immunogenic compositions. One or more immunogenic compositions are selected which appear to have a desired property in any of the aforementioned tests. Further provided is therefore an in vitro method for selecting, from a plurality of immunogenic compositions comprising at least one crossbeta structure and at least one peptide and/or polypeptide and/or protein and/or glycoprotein and/or protein-DNA complex and/or protein-membrane complex and/or lipoprotein with a T-cell epitope or a T-cell epitope motif, one or more immunogenic compositions having a higher chance of being capable of eliciting and/or stimulating a protective prophylactic cellular immune response and/or a therapeutic cellular immune response *in vivo*, as compared to the other immunogenic compositions of said plurality of immunogenic compositions, the method comprising:
selecting, from said plurality of immunogenic compositions, an immunogenic composition:
   - wherein the degree of multimerization of said peptide, polypeptide, protein, glycoprotein, protein-DNA complex, protein-membrane complex and/or lipoprotein in said composition allows recognition, excision, processing and/or presentation of a T-cell epitope of said peptide, polypeptide, protein, glycoprotein, protein-DNA complex, protein-membrane complex and/or lipoprotein by an animal's immune system;
   - wherein between 4-75% of the peptide, polypeptide, protein, glycoprotein, protein-DNA complex, protein-membrane complex and/or lipoprotein content of said composition is in a conformation comprising crossbeta structures;
   - which comprises a crossbeta structure which is capable of specifically binding a crossbeta structure binding compound, preferably tPA, BiP, factor XII, fibronectin, hepatocyte growth factor activator, at least one finger domain of tPA, at least one finger domain of factor XII, at least one finger domain of fibronectin, at least one finger domain of hepatocyte growth factor activator, Thioflavin T, Thioflavin S, Congo Red, CD14, a multiligand receptor such as RAGE or CD36 or CD40 or LOX-1 or TLR2 or TLR4, a crossbeta-specific antibody, preferably crossbeta-specific IgG and/or crossbeta-specific IgM, IgIV, an enriched fraction of IgIV capable of specifically binding a crossbeta structure, Low density lipoprotein Related Protein (LRP), LRP Cluster II, LRP Cluster IV, Scavenger Receptor B-I (SR-BI), SR-A, chrysamine G, a chaperone, a heat shock protein, HSP70, HSP60, HSP90, gp95, calreticulin, a chaperonin, a chaperokine and/or a stress protein; and/or
   - whether a compound capable of specifically binding, recognizing, excising, processing and/or presenting a T-cell epitope of said peptide, polypeptide, protein, glycoprotein, protein-DNA complex, protein-membrane complex and/or lipoprotein is capable of specifically binding, recognizing, excising, processing and/or presenting said T-cell epitope.

In one embodiment it is determined whether a proteolytic system, for example the MHC antigen processing pathway, is capable of recognizing, binding, excising processing and/or presenting a T-cell epitope of said peptide, polypeptide, protein, glycoprotein, protein-DNA complex, protein-membrane complex and/or lipoprotein in the context of either MHC-I and/or MHC-II.

A composition comprising at least one peptide, polypeptide, protein, glycoprotein, protein-DNA complex, protein-membrane complex and/or lipoprotein is provided with at least one crossbeta structure in various ways. In one embodiment said crossbeta structure is induced in at least part of said peptide, polypeptide, protein, glycoprotein, protein-DNA complex, protein-membrane complex and/or lipoprotein. Various methods for inducing a crossbeta structure are known in the art. For instance, said peptide, polypeptide, protein, glycoprotein, protein-DNA complex, protein-membrane complex and/or lipoprotein is at least in part misfolded. In one embodiment, an immunogenic composition comprising at least one peptide, polypeptide, protein, glycoprotein, protein-DNA complex, protein-membrane complex and/or lipoprotein is subjected to a crossbeta inducing procedure. Said crossbeta inducing procedure preferably comprises a change of pH, salt concentration, reducing agent concentration, temperature, buffer and/or chaotropic agent concentration. A method according to the invention, wherein at least one peptide, polypeptide, protein, glycoprotein, protein-DNA complex, protein-membrane complex and/or lipoprotein is subjected to a crossbeta inducing procedure, preferably a change of pH, salt concentration, reducing agent concentration, temperature, buffer and/or chaotropic agent concentration, is therefore also provided. Non-limiting examples of crossbeta inducing procedures are heating, chemical treatments with e.g. high salts, acid or alkaline materials, pressure and other physical treatments. A preferred manner of introducing crossbeta structures in an antigen is by one or more treatments, either in combined fashion or sequentially, of heating, freezing, reduction, oxidation, glycation pegylation, sulphatation, exposure to a chaotropic agent (the chaotropic agent preferably being urea or guanidinium-HCl), phosphorylation, partial proteolysis, chemical lysis, preferably with HCl or cyanogenbromide, sonication, dissolving in organic solutions, preferably 1,1,1,3,3,3-hexafluoro-2-propanol and trifluoroacetic acid, or a combination thereof.

In a particularly preferred embodiment, said immunogenic composition comprising at least one peptide, polypeptide, protein, glycoprotein, protein-DNA complex, protein-membrane complex and/or lipoprotein is coupled to a crossbeta comprising compound. For instance, said peptide, polypeptide, protein, glycoprotein, protein-DNA complex, protein-membrane complex and/or lipoprotein is linked to a peptide or protein comprising a crossbeta structure. It is, however, also possible to administer a crossbeta comprising compound to a composition according to the invention, without linking the crossbeta comprising compound to said peptide, polypeptide, protein, glycoprotein, protein-DNA complex, protein-membrane complex and/or lipoprotein. Preferably said crossbeta comprising compound is an otherwise inert compound. *Inert* is defined as not eliciting and/or stimulating an undesired immune response or another unwanted biochemical reaction in a host, at least not to an unacceptable degree, preferably only to a negligible degree.

A crossbeta structure comprising compound may be added to a composition by itself, but it is also useful to use said crossbeta structure comprising compound as a carrier to which elements of the infectious agent(s) and/or antigen(s) of an immunogenic composition according to the invention are linked. This linkage can be provided through chemical linking (direct or indirect) or, for instance, by expression of the relevant antigen(s) and the crossbeta comprising compound as a fusion protein. In both cases linkers between the two may be present. In both cases dimers, trimers and/or multimers of the antigen (or one or more epitopes of a relevant antigen) may be coupled to a crossbeta comprising compound. However, normal carriers comprising relevant epitopes or antigens coupled to them may also be used. The simple addition of a crossbeta comprising compound will enhance the immunogenicity of such a complex. This is more or less generally true. An immunogenic composition according to the invention may typically comprise a number or all of the normal constituents of an immunogenic composition (in particular a vaccine), supplemented with a crossbeta structure (conformation) comprising compound.

In a preferred embodiment the crossbeta structure comprising compound is itself a vaccine component (i.e. derived from an infectious agent and/or antigen against which an immune response is desired).

An immunogenic composition according to the invention is preferably used for the preparation of a vaccine. A method according to the invention, further comprising producing a vaccine comprising said selected immunogenic composition, is therefore also herewith provided. Preferably a prophylactic and/or therapeutic vaccine is produced. In one embodiment a subunit vaccine is produced.

In one embodiment, an immunogenic composition which is produced and/or selected with a method according to the invention is used as a vaccine. Preferably, no other carriers, adjuvants and/or diluents are necessary because of the presence of crossbeta structures. However, if desired, such carriers, adjuvants and/or diluents may be administered to the vaccine composition at will. Further provided is therefore a use of an immunogenic composition produced and/or selected with a method according to the invention as a vaccine, preferably as a prophylactic and/or therapeutic vaccine. In one embodiment said vaccine comprises a subunit vaccine.

The invention further provides an immunogenic composition selected and/or produced with a method according to the invention. Said immunogenic composition preferably comprises a vaccine, more preferably a prophylactic and/or therapeutic vaccine. An immunogenic composition according to the present invention is particularly suitable for the preparation of a vaccine for the prophylaxis and/or treatment of a disorder caused by a pathogen, tumor, cardiovascular disease, atherosclerosis, amyloidosis, autoimmune disease, graft-versus-host rejection and/or transplant rejection. A use of an immunogenic composition according to the invention for the preparation of a vaccine for the prophylaxis and/or treatment of a disorder caused by a pathogen, tumor, cardiovascular disease, atherosclerosis, amyloidosis, autoimmune disease, graft-versus-host rejection and/or transplant rejection is therefore also herewith provided.

Further provided are uses of such immunogenic compositions for at least in part preventing and/or counteracting such disorders. One embodiment provides a method for at least in part preventing and/or counteracting a disorder caused by a pathogen, tumor, cardiovascular disease, atherosclerosis, amyloidosis, autoimmune disease, graft-versus-host rejection and/or transplant rejection, comprising administering to a subject in need thereof a therapeutically effective amount of an immunogenic composition according to the invention. Said animal is preferably a human individual.

A method according to the invention is particularly suitable for producing and/or selecting an immunogenic composition with desired, preferably improved, immunogenic properties. It is, however, also possible to perform a method according to the invention for improving existing immunogenic compositions. Further provided is therefore a method for improving an immunogenic composition, the composition comprising at least one peptide, polypeptide, protein, glycoprotein, protein-DNA complex, protein-membrane complex and/or lipoprotein comprising a T-cell epitope and/or a T-cell epitope motif, the method comprising providing said composition with at least one crossbeta structure and determining:
- whether the degree of multimerization of said peptide, polypeptide, protein, glycoprotein, protein-DNA complex, protein-membrane complex and/or lipoprotein in said composition allows recognition, excision, processing and/or presentation of a T-cell epitope of said peptide, polypeptide, protein, glycoprotein, protein-DNA complex, protein-membrane complex and/or lipoprotein by an animal's immune system;
- whether between 4-75% of the peptide, polypeptide, protein, glycoprotein, protein-DNA complex, protein-membrane complex and/or lipoprotein content of said composition is in a conformation comprising crossbeta structures;
- whether said at least one crossbeta structure comprises a property allowing recognition, excision, processing and/or presentation of a T-cell epitope of said peptide, polypeptide, protein, glycoprotein and/or lipoprotein by an animal's immune system; and/or
- whether a compound capable of specifically binding, recognizing, excising, processing and/or presenting a known T-cell epitope of said peptide, polypeptide, protein, glycoprotein, protein-DNA complex, protein-membrane complex and/or lipoprotein is capable of specifically binding, recognizing, excising, processing and/or presenting said T-cell epitope.

In one preferred embodiment a method according to the invention is provided, wherein said T-cell epitope is a CTL epitope. In another preferred embodiment, a method according to the invention is provided, wherein said T-cell epitope is a T-helper cell epitope.

A method according to the present invention is particularly suitable for producing and/or selecting an immunogenic composition which is capable of eliciting and/or stimulating a humoral and/or cellular immune response. For a schematic overview of a humoral and cellular immune response, reference is made to Figure 11. In one embodiment, a method according to the present invention is used for producing and/or selecting an immunogenic composition which is specifically adapted for eliciting and/or stimulating a cellular immune response. In another embodiment, a method according to the present invention is used for producing and/or selecting an immunogenic composition which is specifically adapted for avoiding a cellular immune response. In another embodiment, a method according to the present invention is used for producing and/or selecting an immunogenic composition which is specifically adapted for eliciting and/or stimulating both a cellular and a humoral immune response. In another embodiment, a method according to the present invention is used for producing and/or selecting an immunogenic composition which is specifically adapted for eliciting and/or stimulating a humoral immune response.

In order to produce and/or select a composition comprising a peptide, polypeptide, protein, glycoprotein, protein-DNA complex, protein-membrane complex and/or lipoprotein which is specifically adapted for avoiding a cellular immune response, the invention further provides a method for producing an immunogenic composition comprising at least one peptide, polypeptide, protein, glycoprotein, protein-DNA complex, protein-membrane complex and/or lipoprotein, the method comprising:
- determining whether a peptide, polypeptide, protein, glycoprotein, protein-DNA complex, protein-membrane complex and/or lipoprotein lacks a T-cell epitope motif;
- selecting a peptide, polypeptide, protein, glycoprotein, protein-DNA complex, protein-membrane complex and/or lipoprotein lacking a T-cell epitope motif;
- providing a composition comprising said selected peptide, polypeptide, protein, glycoprotein, protein-DNA complex, protein-membrane complex and/or lipoprotein; and
- providing said composition with at least one crossbeta structure.

In order to produce and/or select a (candidate) composition comprising a peptide, polypeptide, protein, glycoprotein, protein-DNA complex, protein-membrane complex and/or lipoprotein which is specifically adapted for avoiding a cellular immune response, the invention further provides a method for producing an immunogenic composition, comprising determining:
- whether the degree of multimerization of said peptide, polypeptide, protein, glycoprotein, protein-DNA complex, protein-membrane complex and/or lipoprotein in said composition does not, or to an acceptable extent, allow recognition, excision, processing and/or presentation of a T-cell epitope of said peptide, polypeptide, protein, glycoprotein, protein-DNA complex, protein-membrane complex and/or lipoprotein by an animal's immune system;
- whether less than 4% of the peptide, polypeptide, protein, glycoprotein, protein-DNA complex, protein-membrane complex and/or lipoprotein content of said composition is in a conformation comprising crossbeta structures;
- whether said at least one crossbeta structure comprises a property which does not, or to an acceptable extent, allow recognition, excision, processing and/or presentation of a T-cell epitope of said peptide, polypeptide, protein, glycoprotein and/or lipoprotein by an animal's immune system; and/or
- whether a compound capable of specifically recognizing, binding, excising, processing and/or presenting a T-cell epitope of said peptide, polypeptide, protein, glycoprotein, protein-DNA complex, protein-membrane complex and/or lipoprotein is not, or to an acceptable extent, capable of specifically recognizing, binding, excising, processing and/or presenting said T-cell epitope.

Said properties are preferably compared with a reference composition. When at least one of said properties appears to be more favorable as compared to said reference composition, said (candidate) composition is preferably used instead of said reference composition.

In order to produce and/or select an immunogenic composition which is suitable for eliciting a humoral immune response, a method according to the present invention preferably comprises the following step:
- determining whether an antibody or a functional fragment or a functional equivalent thereof, capable of specifically binding an epitope of said peptide, polypeptide, protein, glycoprotein, protein-DNA complex, protein-membrane complex and/or lipoprotein, is capable of specifically binding said immunogenic composition. If said antibody or functional fragment or functional equivalent is capable of specifically binding the resulting immunogenic composition, it shows that said epitope is still available for an animal's immune system.

Said epitope of said peptide, polypeptide, protein, glycoprotein, protein-DNA complex, protein-membrane complex and/or lipoprotein is preferably surface-exposed when said peptide, polypeptide, protein, glycoprotein, protein-DNA complex, protein-membrane complex and/or lipoprotein is in its native conformation so that, after administration to a suitable host, an immune response against the native form of said peptide, polypeptide, protein, glycoprotein, protein-DNA complex, protein-membrane complex and/or lipoprotein is elicited.

A functional fragment of an antibody is defined as a fragment which has at least one same property as said antibody in kind, not necessarily in amount. Said functional fragment is preferably capable of binding the same antigen as said antibody, albeit not necessarily to the same extent. A functional fragment of an antibody preferably comprises a single domain antibody, a single chain antibody, a Fab fragment or a F(ab')₂ fragment. A functional equivalent of an antibody is defined as a compound which is capable of specifically binding the same antigen as said antibody. A functional equivalent for instance comprises an antibody which has been altered such that the antigen-binding property of the resulting compound is essentially the same in kind, not necessarily in amount. A functional equivalent is provided in many ways, for instance through conservative amino acid substitution, whereby an amino acid residue is substituted by another residue with generally similar properties (size, hydrophobicity, etc), such that the overall functioning is likely not to be seriously affected.

The invention is further explained in the following examples. These examples do not limit the scope of the invention, but merely serve to clarify the invention.

### Brief description of the drawings

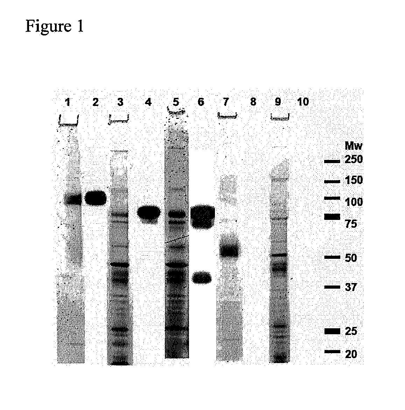

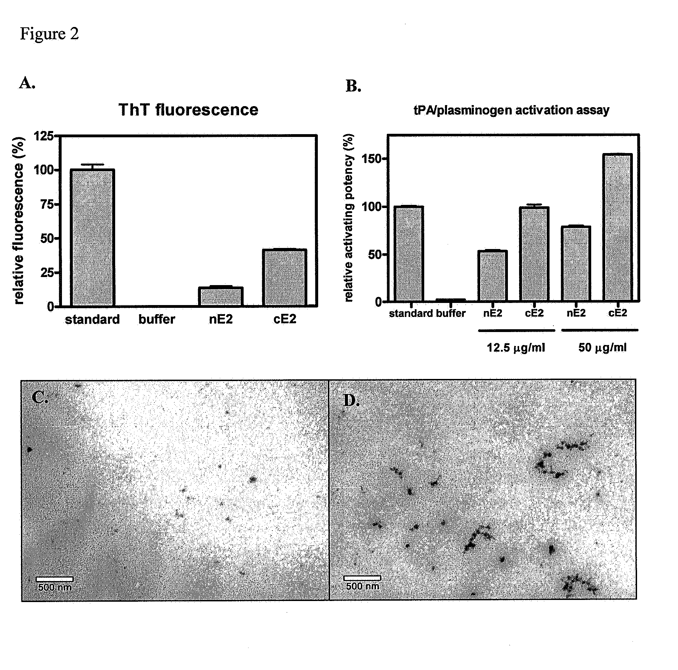

**Figure 1****. Coomassie stained SDS-PA gel and Western blot with nE2 and nE2-FLAG-His.**
   Lane 1: Coomassie nE2-FLAG-His (non-reducing)
   Lane 2: Western blot nE2-FLAG-His (non-reducing; anti-FLAG antibody)
   Lane 3: Coomassie nE2 in culture medium (non-reducing)
   Lane 4: Western blot nE2 in culture medium (non-reducing; mix of 3 monoclonal antibodies)
   Lane 5: Coomassie nE2 dialysed to PBS and concentrated (non-reducing)
   Lane 6: Western blot nE2 dialysed to PBS and concentrated (non-reducing; mix of 3 monoclonal antibodies)
   Lane 7: Coomassie nE2-FLAG-His (reducing)
   Lane 8: Western blot nE2-FLAG-His (reducing; anti-FLAG antibody)
   Lane 9: Coomassie nE2 in culture medium (reducing)
   Lane 10: Western blot nE2 in culture medium (reducing; mix of 3 monoclonal antibodies)
   Lane 11: molecular weight marker
**Figure 2****. Structure analyses of non-treated E2 and misfolded E2. E2 expressed** in Sf9 cells and in cell culture medium was dialyzed against PBS and approximately tenfold concentrated, designated as nE2. Misfolded crossbeta E2 (cE2) was obtained by cyclic heating of nE2 (see text for details). A. Thioflavin T fluorescence enhancement assay with nE2 and cE2 at 100 µg/ml. Standard is 100 µg/ml dOVA. The fluorescence measured with dOVA standard is arbitrarily set to 100%. Buffer control was PBS. B. tPA/plasminogen chromogenic activation assay with nE2 and cE2 at 12.5 and 50 µg/ml in the assay. C. Transmission electron microscopy image of nE2. The scale bar is given in the image. D. TEM image of cE2.

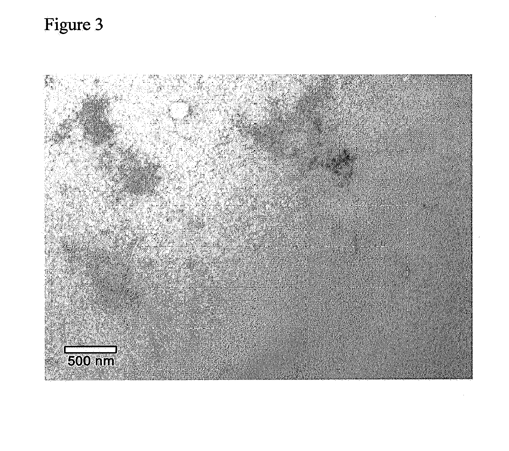

**Figure 3****. Transmission electron microscopy image of misfolded ovalbumin at 1 mg/ml.**

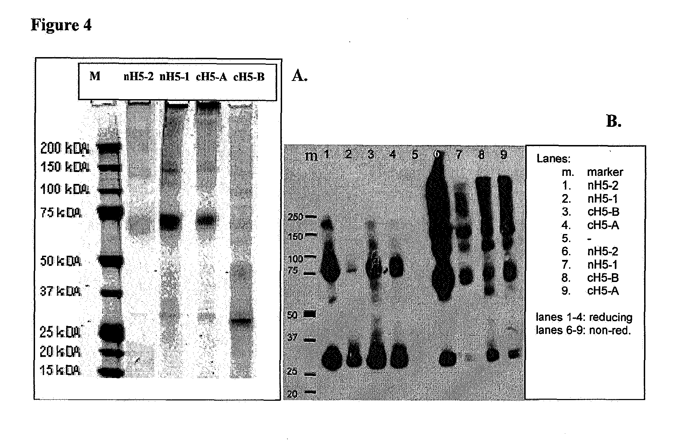

**Figure 4****. Coomassie-stained gel and Western blot with the H5 variants nH5-1, nH5-2, cH5-A, cH5-B. A.** Non reducing SDS NuPage gel applied with nH5-1, nH5-2, cH5-A, cH5-B originating from H5-FLAG-His of H5N1 strain A/HK/156/97. Marker: 6 µl/lane, Precision Plus Protein Dual Color Standards, BioRad, Cat.# 161-0374. Gel:NuPage 4-12% Bis-Tris Gel, 1.0 mm X 10 well, Invitrogen, Cat.# NP0321BOX. M = Marker; nH5-1, 2 µg; nH5-2, 0.66 µg; cH5-A, 2 µg; cH5-B, 2 µg. B. Western blot with the H5 variants nH5-1, nH5-2, cH5-A, cH5-B, analyzed with peroxidase-labeled anti-FLAG antibody. In each indicated lane 30 ng H5 is loaded. H5 is of H5N1 strain A/Hong kong/156/97. Marker: 6 µl/lane, Precision Plus Protein Dual Color Standards, BioRad, Cat.# 161-0374. Gel:NuPage 4-12% Bis-Tris Gel, 1.0 mm X 10 well, Invitrogen, Cat.# NP0321BOX.

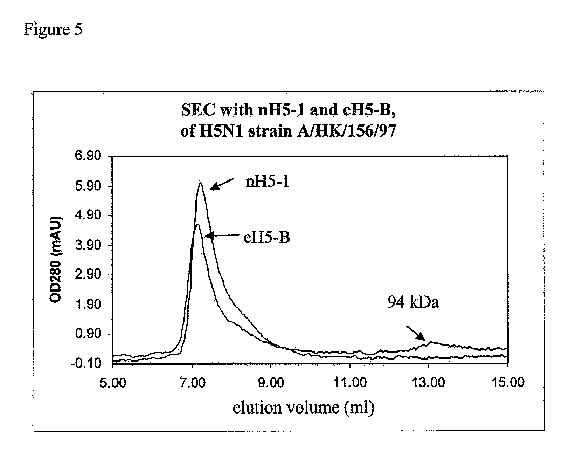

**Figure 5****. Size exclusion chromatography analysis with non-treated H5 and H5 subjected to a misfolding procedure**. The non-treated H5-FLAG-His, nH5-1, and this sample incubated at 37°C with 100 mM DTT (cH5-B), originating from H5 of H5N1 strain A/HK/156/97, were subjected to a SEC column for analysis of the size distribution of H5 multimers, observed on Coomassie-stained SDS-PA gels applied with non-reducing conditions.

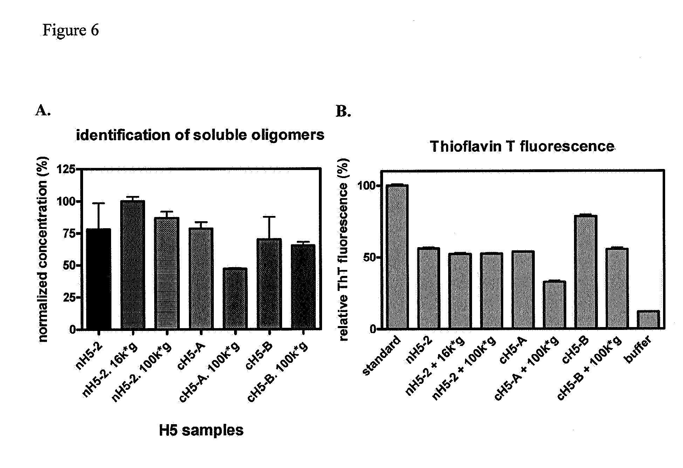

**Figure 6****. Identification of soluble oligomers in H5 samples, of H5N1 strain A/HK/156/97, using ultracentrifugation.** The H5 samples originating from H5N1 strain A/HK/156/97, as indicated in the graphs, were subjected to centrifugation for 10 minutes at 16,000*g (nH5-2, indicated with '16k*g'), or for 60 minutes at 100,000*g (nH5-2, cH5-A, cH5-B, indicated with '100k*g'). A. Protein concentration in the three H5 samples before and after centrifugation at the indicated times/g-forces. Relative concentrations are given for comparison. **B.** ThT fluorescence of four-fold diluted H5 samples before and after centrifugation at the indicated times/g-forces.

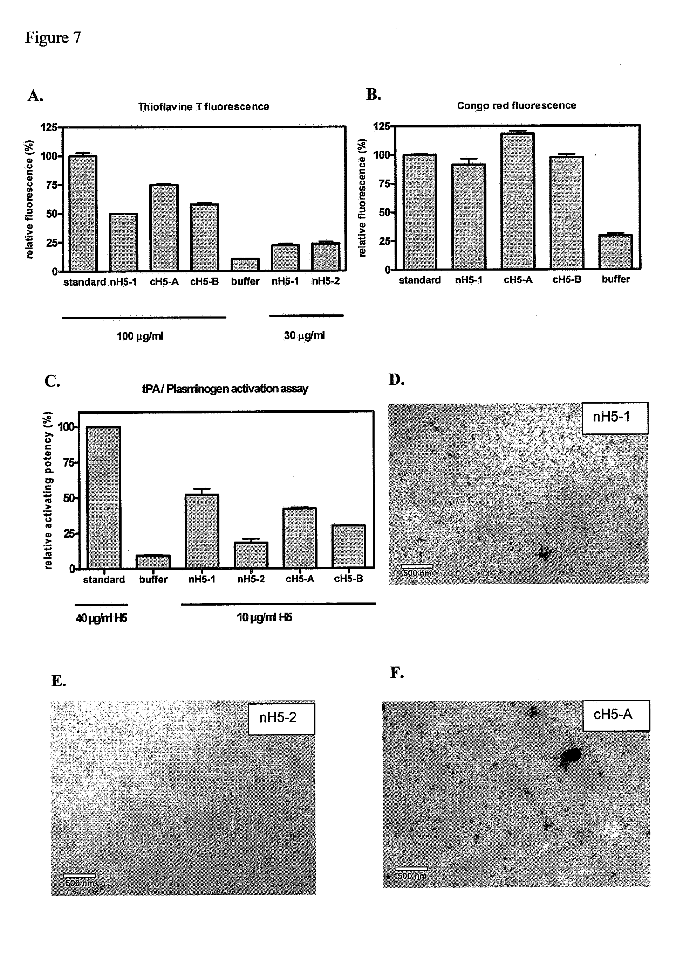

**Figure 7****. Analysis of crossbeta structure in H5-FLAG-His samples.** The H5 originates from H5N1 strain A/HK/156/97 and comprises a C-terminal FLAG-tag, followed by a His-tag. **A.** ThT fluorescence of the two non-treated H5 forms (nH5-1, nH5-2) and the two forms obtained after applying different misfolding procedures (cH5-A, cH5-B), tested at the indicated concentrations. Standard: 100 µg/ml crossbeta dOVA; fluorescence arbitrarily set to 100%. **B**. Congo red fluorescence of the non-treated H5 forms nH5-1 and cH5-A, cH5-B, tested at the indicated concentrations. Standard: 100 µg/ml crossbeta dOVA; fluorescence arbitrarily set to 100%. **C**. tPA/plasminogen activation assay using chromogenic plasmin substrate and depicted H5 solutions at the indicated concentrations. Standard: 40 µg/ml crossbeta dOVA; activity arbitrarily set to 100%. **D**. Transmission electron microscopy image of non-treated H5 form nH5-1. The bar indicates the scale of the image. **E**. Transmission electron microscopy image of nH5-2. **F**. Transmission electron microscopy image of cH5-A obtained after applying a misfolding procedure, as indicated in the text.

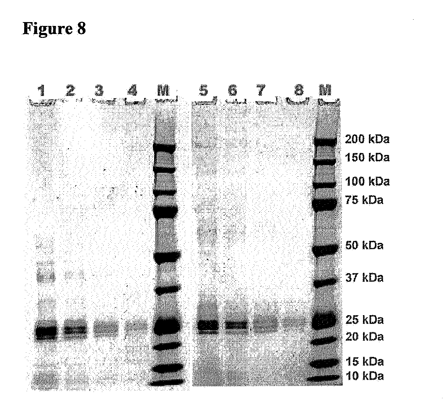

**Figure 8****. Coomassie stained gel with a concentration series of H5 of H5N1 A/Vietnam/1203/04, under reduced and non-reduced conditions.** H5 protein of H5N1 A/VN/1203/04 under reducing (sample 1-4) and non-reducing (sample 5-8) conditions. M = marker, lane 1, 5 = 4 µg H5, lane 2, 6 = 2 µg H5, lane 3, 7 = 1 µgH5, lane 4, 8 = 0.5 µg H5. Marker: 6 µl/lane, Precision Plus Protein Dual Color Standards, BioRad, Cat.# 161-0374. Gel:NuPage 4-12% Bis-Tris Gel, 1.0 mm X 10 well, Invitrogen, Cat.# NP0321BOX.

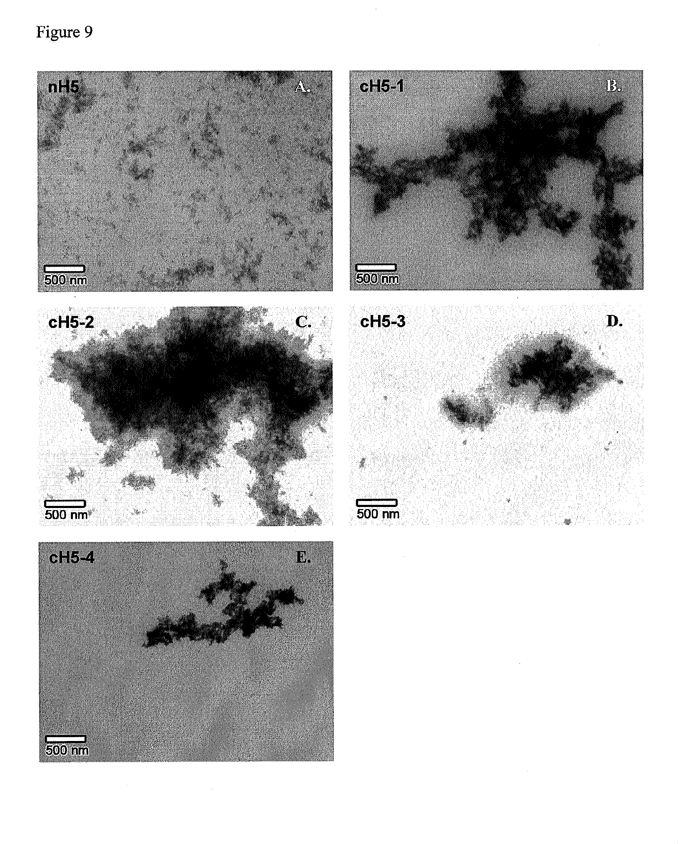

**Figure 9****. TEM images of non-treated H5 of H5N1 A/VN/1203/04, and accompanying misfolded H5 variants cH5-1 - 4, comprising crossbeta.** TEM analysis of nH5 (**A**.) shows amorphous aggregates. The incidence of aggregates is reduced to ∼ 5 aggregates / mesh in cH5-1 (**B**.), but the aggregates are larger in size, more dense and the morphology is changed compared to nH5. A high incidence of dense aggregates was observed in cH5-2 (**C**.). In the preparation of cH5-3 (**D**.), aggregates of similar morphology compared to cH5-2 were observed, but with reduced incidence. Lower aggregate count and dissimilar morphology of aggregates was observed for cH5-4 (**E**.).

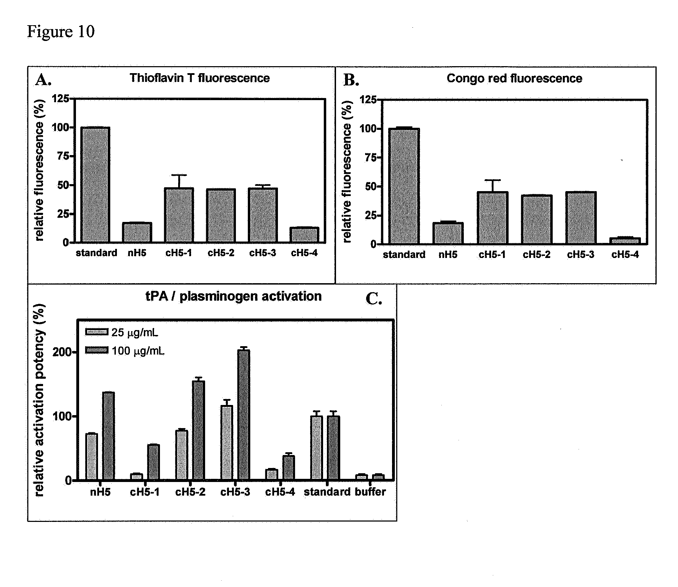

**Figure 10****. ThT and Congo red fluorescence enhancement measurements for non-treated and misfolded H5 (recombinantly produced H5 of H5N1 strain A/Vietnam/1203/04).** Thioflavin T (**A**.) and Congo red fluorescence enhancement measurements (**B**.) of H5 show elevated fluorescence for the preparations cH5-1, cH5-2 and cH5-3 that were subjected to conditions favoring protein misfolding. Reduction in fluorescence intensity was observed in preparation cH5-4. The preparation cH5-1 was slightly turbid with some visible precipitates after heat treatment, which could explain the high standard deviation. **C**. tPA mediated plasminogen activation assay of non-treated and misfolded H5 variants originating from recombinantly produced H5 of H5N1 strain A/VN/1203/04. cH5-2 (150% of standard) and cH5-3 (200% of standard) are more potent cofactors for the activation of tPA/plasminogen compared to the starting material of nH5 (140% of standard). Lower activations were observed with cH5-1 (50% of standard) and cH5-4 (37% of standard) compared to the starting material. Substantial activation is observed with the starting material nH5, indicating that this H5 preparation already harbors misfolded proteins to some extent.
**Figure 11****. Schematic overview of humoral immune response and cellular immune response**

### Examples

### abbreviations

AFM, atomic force microscopy; ANS, 1-anilino-8-naphthalene sulfonate; aPMSF, 4-Amidino-Phenyl)-Methane-Sulfonyl Fluoride; BCA, bicinchoninic acid; bis-ANS, 4,4'-dianilino-1,1'-binaphthyl-5,5'- disulfonic acid; CD, circular dichroism; CR, Congo red; CSFV, Classical Swine Fever Virus; DLS, dynamic light scattering; DNA, Deoxyribonucleic acid; dOVA, misfolded ovalbumin comprising crossbeta; ELISA, enzyme linked immuno sorbent assay; ESI-MS, electron spray ionization mass spectrometry; FPLC, fast protein liquid chromatography; g6p, glucose-6-phosphate; GAHAP, alkaline-phosphatase labelled goat anti-human immunoglobulin antibody; h, hour(s); H#, hemagglutinin protein of influenza virus, number #; HBS, HEPES buffered saline; HCV, hepatitis C virus; HGFA, Hepatocyte growth factor activator; HK, Hong kong; HPLC, high performance, or high-pressure liquid chromatography; HRP, horseradish peroxidase; hrs, hours; Ig, immunoglobulin; IgG, immunoglobulin of the class 'G; IgIV, immunoglobulins intravenous; kDa, kilo Dalton; LAL, Limulus Amoebocyte Lysate; MDa, mega Dalton; NMR, nuclear magnetic resonance; OVA, ovalbumin; PBS, phosphate buffered saline; Plg, plasminogen; RAGE, receptor for advanced glycation end-products; RAMPO, peroxidase labelled rabbit anti-mouse immunoglobulins antibody; RNA, ribonucleic acid; RSV, respiratory syncytial virus; RT, room temperature; SDS-PAGE, sodium-dodecyl sulphate-polyacryl amide gel electrophoresis; SEC, size exclusion chromatography; SWARPO, peroxidase labelled swine anti-rabbit immunoglobulins antibody; TEM, transmission electron microscopy; ThS, Thioflavin S; ThT, Thioflavin T; tPA, tissue type plasminogen activator; VN, Vietnam; W, tryptophan.

### Activation of T-cells

### Analysis of (primary) T cell responses by immunogenic compositions comprising amino-acid sequences with crossbeta conformation

### Isolation and culture of T cell populations.

The ability of immunogenic compositions comprising amino-acid sequences with crossbeta conformation, referred to as 'crossbeta-antigens', to induce (primary) T cell responses in vivo is preferably tested in vitro using T cells isolated from immunized animals, for example mammals. For example, T cells are isolated from mice or from a human individual. Alternatively, activation of naïve T cells is analyzed upon isolation of T-cells from non-immunized animals, for example mammals, for example from mice or human individuals.

Several methods for T-cell isolation are known and commonly used in practice by persons skilled in the art. Preferably, T cells are isolated from blood or splenocytes, for example from splenocytes isolated from immunized mammals, for example mice. Mammals, for example mice are immunized with antigen, preferably immunogenic compositions comprising crossbeta adjuvant and peptide, polypeptide, protein, glycoprotein, protein-DNA complex, protein-membrane complex and/or lipoprotein comprising at least one T-cell epitope motif, preferably once or twice, and cells are isolated preferably between 3 and 14 days after immunization. Preferably, spleen cell suspensions or peripheral blood mononuclear cells are used. Splenocytes are preferably isolated using cell strainers, preferably with a pore size of 100 µm. Preferably, erythrocytes are removed from the cell suspension, preferably by a centrifugation step using Ficoll, or by hemolysis, preferably with a hypotonic buffer, preferably composed of ammonium chloride, preferably at 0.15 mM, and potassium bicarbonate, preferably at 0.1 mM, and ethylendiaminetetaacetic acid, preferably at 0.01 mM.

Subsequently, isolated and washed T-cells are used either directly for analysis of their response towards immunogenic compositions comprising crossbeta adjuvant and peptide, polypeptide, protein, glycoprotein, protein-DNA complex, protein-membrane complex and/or lipoprotein comprising at least one T-cell epitope motif or the isolated and washed T-cells are cultured in appropriate cell culture medium, preferably Dulbecco's Modified Eagle's Medium (DMEM) or RPMI, supplemented with 10% fetal calf serum or human serum, L-glutamine, penicillin, streptomycin and β-mercaptoethanol, and in appropriate cell culture flasks, for example 96-wells or 24-wells culture systems at appropriate cell density, preferably approximately 5 to 35x10⁶ cells per ml. For example, such analyses are performed in an indirect way with antigen presenting cells included in the analysed cell cultures, and/or directly by assessing responsiveness towards T-cell epitope motifs, for example using peptides of such motifs.

### Analysis of T cell response

The number of antigen specific T cells is preferably measured directly, preferably using staining with pre-labeled tetrameric or pentameric MHC molecules, loaded with peptides derived from the antigen, i.e. T-cell epitope motifs, using a FACS apparatus. Preferably, between 5x10⁵ and 5x10⁶ cells are measured. In addition, the following T cell responses are preferably measured: cytokine production, T cell proliferation and cytotoxic activity of CD8⁺ T cells. For analysis of cytokines isolated cells are preferably cultured for 16 to 48 hrs in the presence of antigen, for example as an immunogenic compositions comprising crossbeta adjuvant and peptide, polypeptide, protein, glycoprotein, protein-DNA complex, protein-membrane complex and/or lipoprotein comprising at least one T-cell epitope motif, when antigen presenting cells are included in the analysed cell cultures, or in the presence of T-cell epitope motifs, when cultures of T-cells only are assessed. Preferably a concentration series of immunogenic composition comprising crossbeta and T-cell epitope motif(s), and/or (a) peptide(s) with (an) amino acid sequence(s) of (a) T-cell epitope motif(s) is tested, preferably at concentrations between 10 ng to 500 µg/ml. For example, such crossbeta antigen is provided in the presence of heat shock proteins, such as hsp90, and/or in the presence of a selection of human antibodies, preferably a collection of IVIg, preferably a collection of IVIg selected by a method to enrich for antibodies directed towards crossbeta comprising molecules. Induction of cytokine production is preferably measured using a capture method, i.e. using bi-specific antibodies that bind to a common surface molecule on T-cells and to the cytokine to be analyzed on a FACS apparatus. Preferably interferon-γ (IFN-γ), IL-4 and IL-5 are measured and preferably T-cells are co-stained with antibodies for CD4⁺ and CD8⁺, respectively in order to distinguish the phenotype of the responding T cells. Alternatively, cytokine production is for example measured using ELISPOT analysis or ELISA. T cell proliferation is measured for example using ³H-Thymidine incorporation. Preferably proliferation is analyzed after 5-6 days of culture in the presence of antigen, for example provided as immunogenic compositions comprising crossbeta adjuvant and peptide, polypeptide, protein, glycoprotein, protein-DNA complex, protein-membrane complex and/or lipoprotein comprising at least one T-cell epitope motif, when antigen presenting cells are included in the analysed cell cultures, or in the presence of T-cell epitope motifs, when cultures of T-cells only are assessed, referred to jointly as 'antigen' for the two combined possibilities. Preferably a concentration series of such antigen is tested, preferably at concentrations between 10 ng to 500 µg/ml. Preferably the cells are pulsed with, preferably 0.5 µCi/50 µl ³H-Thymidine for the final 6 to 24 hours. Alternatively, proliferation is measured using BrdU or CSFE. For measurement of cytotoxic activity splenocytes isolated from syngeneic animals are for example used as target cells. Target cells are preferably prepared using antigen, for example immunogenic compositions comprising crossbeta adjuvant and peptide, polypeptide, protein, glycoprotein, protein-DNA complex, protein-membrane complex and/or lipoprotein comprising at least one T-cell epitope motif, when antigen presenting cells are included in the analysed cell cultures, or using peptides of T-cell epitope motifs, for 16-48 hr or 1-4 hours, respectively, and loaded with ⁵¹Cr. Preferably a concentration series of such antigen is tested, preferably at concentrations between 10 ng to 500 µg/ml. After removal of free ⁵¹Cr by washing preferably around 3000 cells are used in a 96 well cluster. Lysis of target cells is measured by the release ⁵¹Cr of following the addition of responder cells, derived from the splenocytes stimulated with antigen, for example immunogenic compositions comprising crossbeta adjuvant and peptide, polypeptide, protein, glycoprotein, protein-DNA complex, protein-membrane complex and/or lipoprotein comprising at least one T-cell epitope motif, or with peptides of T-cell epitope motifs. Preferably a titration of responder cells is tested in ratios of preferably 1:1 to 1:40 with target cells. Alternatively, other target cells, such as tumor cells are for example used, for example E.G7-OVA cells or tumor cells, such as B lymphoma's that can be triggered to present peptides.

For example, mice are immunized with an immunogenic composition comprising ovalbumin as the crossbeta-adjuvated antigen comprising T-cell epitope motifs. Alternatively, human FVIII, E2 derived from classical swine fever virus (CSFV), H5 from influenza virus H5N1 strain A/VN/1203/04 or strain A/HK/156/97, or another protein is used in immunogenic compositions comprising crossbeta adjuvant and T-cell epitope motifs, for example. A crossbeta adjuvant protein is the source of T-cell epitope motifs, and/or a crossbeta adjuvant protein is coupled to an antigen and/or coupled to (a) peptide(s). Preferably, the coupled antigen and/or the coupled peptide(s) are known to be able to generate a T cell response, and/or are predicted to be able to generate a T cell response, preferably by using algorithms and computer based analysis, for example using software such as BIMAS, SYFPEITHI or RANKPEP. For example, such peptides are derived from pathogens, for example from the proteins of influenza virus, for example from H5N1, for example from the nucleoprotein or for example from proteins of human immunodeficiency virus (HIV), plasmodium falciparum, mycobacterium tuberculosis. Such examples include, but are by no means restricted to, peptide AMQMLKETI of the gag24 protein of HIV, and peptides IYSTVASSL, LYQNPTTYI, TYISVGTST, KYVKSNRLV, DYEELKHLL, SYNNTNQEDL, TYISVGTSTL, and KYVKSNRLVL of influenza virus, and in general any known or predicted peptide is used and mixed or coupled with the crossbeta-adjuvated protein. Alternatively, such peptides spanning T-cell epitope motifs are derived from antigens known or predicted to be targets in immunotherapy for cancer or other (human) disease, such as atherosclerosis.

Alternative to primed T cells isolated from immunized non-human animals, or humans which had previously been exposed to an antigen of interest, T cells derived from transgenic animals or T cell clones are for example used. For example, OT-I, OT-II, RF33 or D011.10 cells are used, T cells that are specific for peptides derived from ovalbumin presented in the context of specific MHC class I or MHC class II molecules, respectively peptide SIINFEKL (amino acid residues 257-264) and MHC class I allele Kb for RF33, peptide VAAHAEINEA (327-337) and MHC class II allele IAd for D011.10, peptide SIINFEKL (amino acid residues 257-264) and MHC class I allele Kb for OT-I, peptide AAHAEINEAG (328-338) and MHCII allele IAb for OT-II. Alternatively, one of the T cell hybridoma's B3Z, B)97.10 or 54.8 is for example used. Alternative to splenocytes or monocytes as source of antigen presenting cells, cell lines are for example used as antigen presenting cells, such as for example D1 or DC2.4. Alternative to *in vivo* primed T cells, naive T cells are for example used in cultures comprising antigen presenting cells and/or in cultures with T-cell only, to analyse the ability of immunogenic compositions comprising crossbeta-adjuvated antigen and T-cell epitope motifs, or of peptides spanning T-cell epitope motifs,to activate the T-cells, respectively. Since the number of T cells specific for the peptides spanning T-cell epitope motifs is low the isolated cells are preferably cultured in the presence of mature antigen presenting cells and immunogenic compositions comprising crossbeta-adjuvated antigen and T-cell epitope motifs for preferably around 1 week and subsequently for a prolonged period, preferably several weeks and preferably in the presence of several cytokines, preferably IL-2, PGE2, TNFα and IL-6 to induce optimal expansion of antigen specific T cells. After expansion, T cells are triggered with peptides spanning T-cell epitope motifs for preferably 1 to 6 days and analyzed, preferably as described above for primed T cells, for the production of cytokines and/or for their ability to proliferate in response to specific peptides spanning T-cell epitope motifs.

### Analysis of efficacy of immunogenic compositions comprising T-cell epitope motifs and crossbeta adjuvant in vivo.

Immunizations using immunogenic compositions comprising T-cell epitope motifs and crossbeta adjuvant are preferably aimed at inducing protection against a challenge with a pathogen, and/or aimed at treating a disease. Preferably, the capacity of crossbeta adjuvant protein to induce an effective immune response is analyzed in *vivo.* For example, non-human animals are immunized with immunogenic compositions comprising T-cell epitope motifs and crossbeta adjuvant to induce protection against a challenge with a pathogen, for example a virus, bacteria or parasite. For example, non-human mammals are immunized with immunogenic compositions comprising T-cell epitope motifs and crossbeta adjuvant, comprising for example H5 and/or peptides thereof, and are subsequently challenged with influenza virus. For example, such challenge is with strain A/HK/156/97 or A/VN/1203/04. In another example, pigs are immunized with immunogenic compositions comprising T-cell epitope motifs and crossbeta adjuvant, comprising E2 protein and/or peptides thereof, and or another protein derived from the sequences of the genes encoding proteins of Classical Swine Fever Virus, and challenged with Classical Swine Fever Virus, for example of strain Brescia 456610. Effectiveness of immunization with immunogenic compositions comprising T-cell epitope motifs and crossbeta adjuvant, for the treatment of a disease, for example cancer, when for example a tumor antigen is incorporated in the immunogenic composition, or for example atherosclerosis, is preferably analyzed in immunized mammals. For example an effective immune response is determined by performing an in vivo tumor experiment. For example this is performed using an immunogenic composition comprising ovalbumin as the crossbeta-adjuvated antigen comprising T-cell epitope motifs as antigen and ovalbumin expressing tumor cells, for example E.G7 cells. After immunization with the immunogenic composition as described, after preferably 7 days, animals are injected intradermally in the back with 5x10⁵ E,G7 tumor cells, which were washed preferably in PBS before injection, preferably in a volume of 200 µl. The mice are then examined in time to monitor tumor growth. The tumor growth is preferably estimated by determining the largest and smallest diameters of the tumors and calculating their size. In another example, the mammals are immunized with immunogenic compositions comprising T-cell epitope motifs and crossbeta adjuvant with proteins comprising amino-acid sequences of human papilomavirus proteins (HPV), preferably from the E6 or E7 protein, and challenged with HPV. In another example, the mammals, preferably mammals suffering from atherosclerosis, preferably mice or human, are immunized with immunogenic compositions comprising T-cell epitope motifs and crossbeta adjuvant, for example oxidized LDL and/or glycated protein, for example glycated albumin, and analyzed for progression of diseases, preferably by measuring the size of the atherosclerotic plaque, by determining cytokine levels and/or by scoring survival rates.

### A surrogate marker for T-cell activation in mice in vivo: determination of IgG1/IgG2a titer ratio

As a surrogate marker for the occurrence of a T-cell activation *in vivo* upon subjecting an animal, for example a mouse, to immunizations with an immunogenic composition comprising crossbeta and T-cell epitope motifs, titers of IgG1 and IgG2a are preferably determined using an ELISA with immobilized antigen and dilution series of immune serum, according to methods and protocols known to a person skilled in the art. Increase in IgG1 titers, when compared to pre-immune serum and/or serum of the animal(s) that received placebo, is an indicative measure for the occurrence of a T-helper 2 mediated humoral response, with activation of CD4+ T-helper cells. Increase in IgG2a titers, when compared to pre-immune serum and/or serum of the animal(s) that received placebo, is an indicative measure for the occurrence of a T-helper 1 mediated cellular immune response, with activation of CD8+ cytotoxic T-cells. In addition, total IgG titers are determined as a indicative measure for activation of CD4+ positive T-helper cells.

### T-cell activation: summary

Disappearing Epitope Scanning Technology of this Example comprises two main approaches resulting in the ability of selecting from a plurality of immunogenic compositions those immunogenic compositions having a greater chance of being capable of eliciting and/or stimulating a protective prophylactic immune response and/or a therapeutic immune response *in vivo,* as compared to the other immunogenic compositions of a plurality of immunogenic compositions. The elicited immune response comprises activation of T-cells, for example resulting in a CD4+ T-help response, and/or resulting in a CD8+ cytotoxic T-lymphocyte response. When T-cell epitope motifs are not known for an antigen and/or when T-cell epitope motifs are not adequately or not at all predicted by algorithms and computer based analysis, approach I is preferred:

### Approach I. Design of immunogenic compositions comprising one or more T-cell epitope motifs and crossbeta adjuvant, checked for functionality with cell cultures of APCs + naïve and/or primed T-cells.

When applying approach I. of the Disappearing Epitope Scanning Technology, one predicted and/or putative T-cell epitope motif and/or series of predicted and/or putative motifs are incorporated in immunogenic compositions comprising crossbeta adjuvant. Putative T-cell epitope motifs are for example obtained by synthesizing peptides covering overlapping sequences of the antigen, comprising preferably the number of amino-acid residues known to be required for presentation by major histocompatibility complexes, for example 5-30 amino-acid residues. The sequence overlap between two adjacent peptides is for example 1-10 amino-acid residues.

When T-cell epitope motifs are known and/or when algorithms and computer based analysis predict T-cell epitope motifs accurately to a large extent, approach II of the Disappearing Epitope Scanning Technology is preferred:

### Approach II. Design of ready-to-use immunogenic compositions comprising one or more known and/or predicted T-cell epitope motifs and crossbeta adjuvant.

### Peptides spanning T-cell epitope motifs are

1. predicted T-cell epitope motifs (MHC class I restricted or MHC class II restricted) obtained using prediction programs, and/or are
2. known T-cell epitope motifs, like for example, but not limited to, those identified for H5 or OVA.

***The known and*/*or predicted T-cell epitope motifs are***
i. part of the crossbeta-adjuvated antigen comprising the motifs, and/or are
ii. part of a natively folded antigen comprising the motifs, that is
   a. coupled and/or mixed with crossbeta-adjuvated antigen comprising the motifs, and/or that is
   b. coupled and/or mixed with crossbeta-adjuvated protein with unrelated amino-acid sequence with respect to the amino-acid sequence of the parent antigen from which the peptides are derived,
   for use as an immunogenic composition in vivo, as a vaccine candidate preceding a challenge with tumor cells or pathogen, and/or with the purpose to obtain primed T-cells, and/or for use as an immunogenic composition in vitro for assessing T-cell activation in vitro, by using co-cultures of APCs and naïve and/or primed T-cells, and/or T-cell clones specific for a known T-cell epitope motif, and/or
iii. used as sole peptides
   a. having conformations covering those folds that are present when the peptides are presented by major histocompatibility complexes at APCs,
      for assessing direct stimulation of cultured naïve and/or primed T-cells, and/or T-cell clones specific for a known T-cell epitope motif, in the presence of the selected major histocompatibility complexes, or
   b. comprising crossbeta conformation for 4-75%, and/or
   c. coupled to and/or mixed with crossbeta-adjuvated antigen comprising the motifs, and/or
   d. coupled to and/or mixed with crossbeta-adjuvated protein with unrelated amino-acid sequence with respect to the amino-acid sequence of the parent antigen from which the peptides are derived,
for assessing T-cell activation in vivo upon immunization, and/or for obtaining primed T-cells upon immunizations, and/or for assessing T-cell activation in vitro, by using co-cultures of APCs and naive and/or primed T-cells and/or T-cell clones specific for a known T-cell epitope motif.

Animal or human individuals that have T-cell clones specific for T-cell epitope motifs under investigation, upon previous immunization with an antigen comprising T-cell epitope motifs, for example upon vaccination and/or for example upon suffering and subsequent recovering from an infection, are serving as a source of T-cells used for the aforementioned experiments comprising cultured primed T-cells.

### Detection of proteins comprising crossbeta

### Protein misfolding and crossbeta structure

Several techniques are generally available by a person skilled in the art to analyze the presence of crossbeta, i.e. non-native structural elements in unfolded proteins, misfolded proteins and multimerized forms thereof. For example, and as described in more detail below, these techniques allow the detection of non-native epitopes, the detection of the size of the misfolded proteins and multimers thereof and the analysis of the shape of the aggregates. Combined, these techniques allow detailed description of the presence and characteristics of proteins comprising crossbeta. Therefore these techniques allow the description of immunogenic compositions comprising crossbeta. Preferably, when applying any of the techniques described below, a reference sample of the non-treated protein is compared to the protein that is subjected to misfolding procedures, for comparison.

### Crossbeta detection assays

### Congo red fluorescence

Congo red is a relatively small molecule (chemical name: C₃₂H₂₂N₆Na₂O₆S₂) that is commonly used as histological dye for detection of amyloid. The specificity of this staining results from Congo red's affinity for binding to fibrillar proteins enriched in beta-sheet conformation and comprising crossbeta. Congo red is also used to selectively stain protein aggregates with amyloid properties that do not necessarily form fibrils. Congo red is also used in a fluorescence enhancement assay to identify proteins with crossbeta in solution. This assay, also termed Congo red fluorescence measurement, is for example performed as described in patent application WO2007008072 paragraph [101]. Fluorescence can be read on various readers, for example fluorescence is read on a Gemini XPS microplate reader (Molecular Devices).

### Thioflavin T fluorescence

Thioflavin T, like Congo red, is also used by pathologists to visualize plaques composed of amyloid. It also binds to beta sheets, such as those in amyloid oligomers. The dye undergoes a characteristic 115 nm red shift of its excitation spectrum that may be selectively excited at 442 nm, resulting in a fluorescence signal at 482 nm. This red shift is selectively observed if structures of amyloid fibrillar nature are present. It will not undergo this red shift upon binding to precursor monomers or small oligomers, or if there is a high beta sheet content in a non-amyloid context. If no amyloid fibrils are present in solution, excitation and emission occur at 342 and 430 nm respectively. Thioflavin T is often used to detect crossbeta in solutions. For example, the Thioflavin T fluorescence enhancement assay, also termed ThT fluorescence measurement, are performed as described in patent application WO2007008072, paragraph [101]. Fluoresence can de read on various readers, for example fluorescence is read on a Gemini XPS microplate reader (Molecular Devices).

### Thioflavin S fluorescence

Thioflavin S, is a dye similar to Thioflavin T and the fluorescence assay is performed essentially similar to ThT and CR fluorescence measurements.

### tPA binding ELISA

tPA binding ELISA with immobilized misfolded proteins; is performed as described in patent application WO2007008070, paragraph [35-36]. One of our first discoveries was that tPA binds specifically to misfolded proteins comprising crossbeta. Binding of tPA to misfolded proteins is mediated by its finger domain. Other finger domains and proteins comprising homologous finger domains are also applicable in a similar ELISA setup (see below).

### BiP binding ELISA

BiP binding ELISA with immobilized misfolded proteins; is performed as described in patent application WO2007108675, section "Binding of BiP to misfolded proteins with crossbeta structure", with the modification that BiP purified from cell culture medium using Ni²⁺ based affinity chromatography, is used in the ELISAs. It has been demonstrated previously that chaperones like for example BiP bind specifically to misfolded proteins comprising crossbeta. Other heat shock proteins, such as hsp70, hsp90 are also applicable in a similar ELISA setup.

### IgIV bindig ELISA

Immunoglobulins intravenous (IgIV) binding ELISA with immobilized misfolded proteins; is performed as described in patent application WO2007094668, paragraph [0115-0117]. Alternatively, IgIV that is enriched using an affinity matrix with immobilized protein(s) comprising crossbeta, is used for the binding ELISA with immobilized misfolded proteins (see patent application WO2007094668, paragraph [0143]). It has been demonstrated previously that a subset of immunoglobulins in IgIV bind selectively and specifically to misfolded proteins comprising crossbeta. Other antibodies directed against misfolded proteins are also applicable in a similar ELISA setup.

### Finger binding ELISA using fibronectin finger domains

Fibronectin finger 4-5 binding ELISA with immobilized misfolded proteins; is performed as described in patent application WO2007008072. It has been demonstrated previously that finger domains of fibronectin selectively and specifically bind to misfolded proteins comprising crossbeta. In addition to, or alternative to finger domains of fibronectin, finger domains of tPA and/or factor XII and/or hepatocyte growth factor activator are used.

### Factor XII activation assay

Factor XII / prekallikrein activation assay is performed as described in patent application WO2007008070, paragraph [31-34]. It has been demonstrated previously that factor XII selectively and specifically bind to misfolded proteins comprising crossbeta, resulting in its activation.

### tPA/plasminogen activaction assay

Enhancement of tPA/plasminogen activity upon exposure of the two serine proteases to misfolded proteins was determined using a standardized chromogenic assay (see for example patent application WO2006101387, paragraph [0195], patent application WO2007008070, paragraph [31-34], and [Kranenburg et al., 2002, Curr. Biology 12(22), pp.1833)]. Both tPA and plasminogen act in the Crossbeta Pathway. Enhancement of the activity of the crossbeta binding proteases is a measure for the presence of misfolded proteins comprising crossbeta structure. 4-Amidinophenylmethanesulfonyl fluoride hydrochloride (aPMSF, Sigma, A6664) was added to protein solutions to a final concentration of 1.25 mM from a 5 mM stock. Protein solutions with added aPMSF were kept at 4°C for 16 h before use in a tPA/plasminogen activation assay. In this way, proteases that are putatively present in protein solutions to be analyzed, and that may act on tPA, plasminogen, plasmin and/or the chromogenic substrate for plasmin, are inactivated, to prevent interference in the assay.

### Binding assays

Apart from the above described binding assays using crossbeta binding compounds, additional crossbeta binding compounds are suitable for use in binding assays for determination of the presence and extent of crossbeta in a sample of a peptide, polypeptide, protein, glycoprotein, protein-DNA complex, protein-membrane complex and/or lipoprotein. In general, crossbeta binding compounds useful for these determinations are tPA, BiP, factor XII, fibronectin, hepatocyte growth factor activator, at least one finger domain of tPA, at least one finger domain of factor XII, at least one finger domain of fibronectin, at least one finger domain of hepatocyte growth factor activator, Thioflavin T, Thioflavin S, Congo Red, CD 14, a multiligand receptor such as RAGE or CD36 or CD40 or LOX-1 or TLR2 or TLR4, a crossbeta-specific antibody, preferably crossbeta-specific IgG and/or crossbeta-specific IgM, IgIV, an enriched fraction of IgIV capable of specifically binding a crossbeta structure, Low density lipoprotein Related Protein (LRP), LRP Cluster II, LRP Cluster IV, Scavenger Receptor B-I (SR-BI), SR-A, chrysamine G, a chaperone, a heat shock protein, HSP70, HSP60, HSP90, gp95, calreticulin, a chaperonin, a chaperokine and/or a stress protein. In addition, as disclosed previously in patent application WO2007008072, crossbeta binding compounds for use for the aforementioned determinations are 2-(4'-(methylamino)phenyl)-6-methylbenzothiaziole, styryl dyes, BTA-1, Poly(thiophene acetic acid), conjugated polyeclectrolyte, PTAA-Li , Dehydro-glaucine, Ammophedrine, isoboldine, Thaliporphine, thalicmidine, Haematein, ellagic acid, Ammophedrine HBr, corynanthine, Orcein.

### Measurements of protein refolding and changes in protein conformation & multimer size and multimer size distribution analysis

### Turbidity of protein solutions

With turbidity measurements the diffraction of light scattered by protein particles in the sample is detected. Light is scattered by the solid particles and absorbed by dissolved protein. In a turbidity measurement the amount of insoluble particles in a solution is determined. This aspect is used to determine the amount of insoluble protein in samples of protein that is subjected to misfolding conditions, compared to the fraction of insoluble protein in the non-treated reference sample.

### Recording changes in binding characteristics of binding partners for a protein

Antibodies specific for a protein in a certain conformation are used to measure the amount of this protein present in this specific state. Upon treatment of the protein using misfolding conditions, binding of antibodies is inhibited or diminished, which is used as a measure for the progress and extent of misfolding. In addition or alternatively, antibodies are used that are specific for certain conformations and/or post-translational modifications, for example glycation, oxidation, citrullination (gain of binding to the protein subjected to misfolding conditions). When for example glycation and/or oxidation and/or citrullination procedures is/are part of the misfolding procedure, the effect of the treatment with respect to the occurrence of modified amino-acid residues is recorded by determining the relative binding of the antibodies, compared to the non-treated reference protein. Alternatively or in addition to the use of antibodies, any binding partner and/or ligand of the non-treated protein is used similarly, and/or any binding partner and/or ligand other than antibodies, of the misfolded protein is used. When a protein changes conformation ligands or binding partners express altered binding characteristics, which is used as a measure for the extent of protein modification and/or extent of misfolding. This binding of antibodies, ligands and/or binding partners is measured using various techniques, such as direct and/or indirect ELISA, surface plasmon resonance, affinity chromatography and immuno-precipitation approaches.

### Differential scanning calorimetry/micro DSC for detecting changes in protein conformation

Differential scanning calorimetry (DSC) is a thermo-analytical technique in which the difference in the amount of heat required to increase the temperature of a sample and a reference is measured as a function of temperature. The temperature is linearly increased over time. When the protein in the sample changes its conformation, more or less heat (depending on if it is an endo- or exothermic reaction) will be required to increase the temperature at the same rate as the reference sample. In this way the conformational changes as a result of an increase in temperature can be measured.

### Particle analyzer

A particle analyzer measures the diffraction of a laser beam when targeted at a sample. The resulting data is transformed by a Fourier transformation and gives information about particle size and shape. When applied to protein solutions, putatively present protein aggregates are detected, when larger than the lower detection limit of the apparatus, for example in the sub-micron range.

### Direct light microscope

With a regular direct-light microscope with a preferable magnification range of 10x - 100x, one can determine visually if there are any protein aggregates present in a sample.

### Photon correlation spectroscopy (dynamic light scattering spectroscopy)

Photon correlation spectroscopy can be used to measure particle size distribution in a sample in the nm-µm range.

### Nuclear magnetic resonance spectroscopy

Nuclear Magnetic Resonance Spectroscopy (NMR) can be used to assess the electromagnetic properties of certain nuclei in proteins. With this technique the resonance frequency and energy absorption of protons in a molecule are measured. From this data structural information about the protein, like angles of certain chemical bonds, the lengths of these bonds and which parts of the protein are internally buried, can be obtained. This information can then be used to calculate the complete three dimensional structure of a protein. This method however is normally restricted to relatively small molecules. However with special techniques like incorporation of specific isotopes and transverse relaxation optimized spectroscopy, much larger proteins can now be studied with NMR.

### X-ray diffraction

In X-ray diffraction with protein crystals, the elastic scattering of X-rays from a crystallized protein is measured. In this way the arrangement of the atoms in the protein can be determined, resulting in a three-dimensional structural model of the protein. First a protein is crystallized and then a diffraction pattern is measured by irradiating the crystallized protein with an X-ray beam. This diffraction pattern is a representation of how the X-ray beam is scattered from the electrons in the crystal. By gradually rotating the crystal in the X-ray beam, the different atomic positions in the crystal can be determined. This results in an electron density map, with which a complete three-dimensional atomic model of the crystallized protein can be calculated, regularly at the 1-3 Å scale. In this model it can be deduced whether protein molecules underwent conformational changes upon treatment with misfolding conditions, when compared to the structural model of the non-treated protein. In addition, modifications of amino-acid residues become apparent in the structural model, as well as whether the protein molecule forms ordered multimers of a defined size, like for example in the range of dimers - octamers.
Determination of the presence of crossbeta in fibers comprising crystallites, and/or in other appearances of protein aggregates comprising at least a fraction of the protein molecules in a crystalline ordering, can be assessed using X-ray fiber diffraction, as for example shown in [Bouma et al., J.Biol.Chem. V278, No.43, pp.41810-41819, 2003, "Glycation Induces Formation of Amyloid Crossbeta Structure in Albumin"].

### Fourier Transform infrared spectroscopy

Detection of protein secondary structure in Fourier Transform Infrared Spectroscopy (FTIR), an infrared beam is split in two separate beams. One beam is reflected on a fixed mirror, the second on a moving mirror. These two beams together generate an interferogram which consists of every infrared frequency in the spectrum. When transmitted through a sample specific functional groups in the protein adsorb infrared of a specific wavelength. The resulting interferogram must be Fourier transformed, before it can be interpreted. This Fourier transformed interferogram gives a plot of al the different frequencies plotted against their adsorption. This interferogram is specific for the structure of a protein, like a 'molecular fingerprint', and provides information on types of atomic bonds present in the molecule, as well as the spatial arrangement of atoms in for example alpha-helices or beta-sheets.

### 8-Anilino-1-naphthalenesulfonic acid fluorescence enhancement assay

8-Anilino-1-naphthalenesulfonic acid (ANS) fluorescence enhancement assay, or ANS fluorescence measurement; was performed as described in patent application WO2007094668. Modification: fluorescence is read on a Gemini XPS microplate reader (Molecular Devices).
ANS is a chemical binds to hydrophobic surfaces of a protein and its fluorescence spectrum shifts upon binding. When proteins are in an unfolded state, they generally display more hydrophobic sites, resulting in an increased ANS shift compared to the protein in its native more globular state. ANS can therefore be used to measure protein unfolding.

### bis-ANS fluorescence enhancement assay

4,4' dianilio-1,1' binaphthyl-5,5' disulfonic acid di-potassium salt (Bis-ANS) fluorescence enhancement assay; is performed as described in patent application WO2007094668. Essentially, bis-ANS has characteristics comparable to ANS, and bis-ANS is also used to probe for differences in solvent exposure of hydrophobic patches of proteins, when measuring bis-ANS binding with a reference protein samples, and with a protein sample subjected to a misfolding procedure.

### Gel electrophoresis

Gel electrophoresis using sodium dodecyl-sulphate polyacryl amide gels (SDS-PAGE) and Coomassie stain, with various gels with resolutions between for example 100 Da up to several thousands of kDa, provides information on the occurrence of protein modifications and on the occurrence of multimers. Multimers that are not covalently coupled may also appear as monomers upon the assay conditions applied, i.e. heating protein samples in assay buffer comprising SDS. Samples are heated in the presence or absence of a reducing agent like for example dithiothreitol (DTT), when the protein amino-acid sequence comprises cysteines, that can form disulphide bonds upon subjecting the protein to misfolding conditions.

### Western blot

When antibodies are available that bind to epitopes on the protein under the denaturing conditions as applied during SDS-PAGE, Western blotting is performed with the same protein samples as applied for SDS-PAGE with Coomassie stain, using the same molecular weight cut-off gels, and using the same protein sample handling approaches.

### Centrifugation

Centrifugation and subsequent comparing the protein concentration in the supernatant with respect to the concentration before centrifugation provides insight into the presence of insoluble precipitates in a protein sample. Upon applying increasing g-forces for a constant time, and/or upon applying fixed or increasing g-forces for an increasing time frame, to a protein solution, with analyzing the protein content in between each step, information is gathered about the presence of insoluble multimers. For example, protein solutions are subjected for 10 minutes to 16,000*g, or for 60 minutes to 100,000*g. The first approach is commonly used to prepare protein solutions for, for example use on FPLC columns or in biological assays, with the aim of pelleting insoluble protein aggregates and using the supernatant with soluble protein. It is generally accepted that after applying 100,000*g for 60 minutes to a protein solution, only soluble multimers are left in the supernatant. As multimers ranging from monomers up to huge multimers comprising thousands of protein monomers may all have a density equal to the density of the buffer solution, applying these g-forees to protein solutions does not separate exclusively on size, but on density differences between the solution and the protein multimers.

### Electron spray ionization mass spectrometry

Electron spray ionization mass spectrometry (ESI-MS) with protein solutions provides information on the multimer size distribution when sizes range from tens of Da up to the MDa range.

### Ultrasonic spectrometry

Ultrasonic spectroscopy analysis, for example using an Ichos-II (Process Analysis and Automation, Ltd), provides insight into protein conformation and changes in tertiary structure are measured. In addition the technique can provide information on particle size of protein assemblies, and allows for monitoring protein concentration.

### Dialysis (membranes with increasing molecular weight cut-off)

Using one or a series of dialysis membranes with varying molecular weight cut-offs, size distribution / multimer distribution of protein can be assessed at the sub-oligomer scale, depending on the molecular weight of the monomer. Protein concentration analysis between each dialysis step with gradually increasing pore size (suitable for molecular weight ranges between approximately 1000-50000 Da). Protein concentration is for example monitored using BCA or Coomassie+ determinations (Pierce), and/or absorbance measurements at 280 nm, using for example the nanodrop technology (Attana).

### Filtration (filters with increasing molecular weight cut-off)

Filtration using a series of filters with gradually increasing MW cut-offs, ranging from the monomer size of the protein under investigation up to the largest MW cut-off available, reveals information on the distribution and presence of protein molecules in multimers in the range from monomers, lower-order multimers and large multimers comprising several hundreds of monomers. For example, filters with a MW cut-off of 1 kDa up to filters with a cut-off of 5 µm (MW's for example 1/3/10/30/50/100 kDa, completed with filters with cut-offs of for example 200/400/1000/5000 nm). In between each subsequent filtration step, protein concentration is assessed using for example the BCA or Coomassie+ method (Pierce), and/or visualization on SDS-PA gel stained with Coomassie.

### Transmission electron microscopy

Transmission electron microscopy (TEM is a imaging technique that provides structural information of proteins at a nm to µm scale. With this resolution it is possible to identify the occurrence of protein assemblies ranging from monomers up to multimers of several thousands molecules, depending on the molecular weight of the parent protein molecule. Furthermore, TEM imaging provides insight into the structural appearance of protein multimers. For example, protein multimers appear as rods, globular structures, strings of globular structures, amorphous assemblies, unbranched fibers, commonly termed fibrils, branched fibrils, and/or combinations thereof.

In the current studies, TEM images were collected using a Jeol 1200 EX transmission electron microscope (Jeol Ltd., Tokyo, Japan) at an excitation voltage of 80 kV. For each sample, the formvar and carbon-coated side of a 100-mesh copper or nickel grid was positioned on a 5 µl drop of protein solution for 5 minutes. Afterwards, it was positioned on a 100 µl drop of PBS for 2 minutes, followed by three 2-minute incubations with a 100 µl drop of distilled water. The grids were then stained for 2 minutes with a 100 µl drop of 2% (m/v) methylcellulose with 0.4% uranyl acetate pH 4. Excess fluid was removed by streaking the side of the grids over filter paper, and the grids were subsequently dried under a lamp. Samples were analysed at a magnification of 10K.

### Atomic force microscopy

Similar to TEM imaging, atomic force microscopy provides insights into the structural appearance of protein molecules at the protein monomer level up to the macroscopic level of large multimers of protein molecules.

### Size exclusion chromatography, or gel filtration chromatography

With size exclusion chromatography (SEC) using HPLC and/or FPLC, a qualitative and quantitative insight is obtained about the distribution of protein molecules over monomers up to multimers, with a detectable size limit of the multimers restricted by the type of SEC column that is used. SEC columns are available with the ability to separate molecular sizes in the sub kDa range up to in the MDa range. The type of column is selected based on the molecular weight of the analyzed protein, and on any indicative information at forehand about the expected range of multimeric sizes. Preferably, a reference non-treated protein is compared to a protein that is subjected to misfolding procedures.

### Tryptophan fluorescence

Assessment of differences in tryptophan (W) fluorescence intensity between two appearances of the same protein provides information on the occurrence of protein folding differences. In general, in globular proteins W residues are mostly buried in the interior of the globular fold. Upon unfolding, refolding, misfolding, W residues tend to become more solvent exposed, which is recorded in the W fluorescence measurement as a change in fluorescent intensity compared to the protein with a more native fold.

### Dynamic Light Scattering

With the Dynamic Light Scattering (DLS) technique, particle size and particle size distribution is assessed. When protein solutions are considered distribution of proteins over a range of multimers ranging from monomers up to multimers is measured, with the upper limit of detected multimer size limited by the resolution of the DLS technique.

### Circular dichroism spectropolarimetry

With circular dichroism spectropolarimetry (CD) the relative presence of protein secondary structural elements is determined. Therefore, this technique allows for the comparison of the relative occurrence of alpha-helix, beta-sheet and random coil between a reference protein that is non-treated, and the protein that is subjected to misfolding conditions. An example of a CD experiment for assessment of conformational changes in proteins upon treatment with misfolding conditions is given in [Bouma et al., J.Biol.Chem. V278, No.43, pp.41810-41819, 2003, "Glycation Induces Formation of Amyloid Crossbeta Structure in Albumin"].

### Native gel electrophoresis

Distribution over multimers in the range of approximately monomers up to 100-mers is assessed by applying native gel electrophoresis. For this purpose a reference non-treated protein sample is compared to a protein sample which is subjected to a misfolding procedure. When misfolding procedures are applied that introduce modifications on amino-acid residues, like for example but not limited to, glycation or oxidation or citrullination, these changes are becoming apparent on native gels, as well.

### Examples of proteins that are used for preparation of immunogenic compositions

### Envelope protein E2 of Classical Swine Fever Virus

The envelope protein E2 of Classical Swine Fever Virus (CSFV) strain Brescia 456610 is used as a prototype subunit vaccine candidate for examples described below. Currently, a subunit vaccine that provides protection in pigs against CSF comprises recombinantly produced E2 antigen in cell culture medium, adjuvated with a double emulsion of water-in-oil-in-water, comprising PBS, Marcol 52, Montanide 80. The vaccine comprises at least 32 µg E2/dose of 2 ml, and is injected intramuscularly.

E2 was recombinantly produced in insect Sf9 cells (Animal Sciences Group, Lelystad, The Netherlands) or in human embryonic kidney 293 cells (293) (ABC-Protein Expression facility, University of Utrecht, The Netherlands), as described in patent application WO2007008070. E2 produced in Sf9 cells and lacking any tags is in PBS after dialysis of cell culture medium (storage of aliquots at -20°C or at -80°C), or in cell culture medium (storage at -20°C). Cell culture medium is SF900 II medium with 0.2% pluronic (serum free). After culturing of cells, the cell culture medium is micro-filtrated. Virus is inactivated with 8-12 mM 2-bromo-ethyl-ammonium bromide. The E2 produced in 293 cells comprises a C-terminal FLAG-tag followed by a His-tag, and is purified using Ni²⁺-based affinity chromatography. Concentration and purity of E2 from both sources is determined as follows. Quantification of the total protein concentration is performed with the BCA method (Pierce) or with the Coomassie+ method (Pierce). E2 specific bands on a Western blot are visualized using anti-FLAG antibody (mouse antibody, M2, peroxidase conjugate; Sigma, A-8592) for the E2-FLAG-His construct, and a 1:1:1 mixture of three horseradish peroxidase (HRP) tagged mouse monoclonal anti-E2 antibodies (CediCon CSFV 21.2, 39.5 and 44.3; Prionics Lelystad) for the E2-FLAG-His construct and the E2 construct from Sf9 cells. The purity of E2 batches was determined by densitometry with a Coomassie stained sodium dodecyl sulphate-polyacryl amide (SDS-PA) gel after electrophoresis.

In Figure 1, SDS-PA gels and Western blots with E2 produced in Sf9 cells and E2-FLAG-His produced in 293 cells are shown, with reducing and non-reducing conditions. It is clearly seen that the main fraction of both E2 batches appears as dimers on the gel and blot, when applied with non-reducing sample buffer. Apparently, those dimers are covalently coupled, since treatment of E2 from 293 cells with DTT reveals monomers at the expected molecular weight of approximately 47 kDa. No E2 bands are visualized on the blot when analysing E2 from Sf9 cells under reducing conditions. The observation that E2 appears as at least two monomer and dimer bands is most likely related to the presence of glycosylation isoforms.

Before use in misfolding procedures, crossbeta analyses, multimer analyses and/or immunization, non-treated E2 solution was warmed to 37°C for 10-30 minutes, left on a roller device for 10-30 minutes, at room temperature, warmed again at 37°C for 0-30 minutes and left again on a roller device for 0-30 minutes. Alternatively, non-treated E2 solutions were quickly thawed at 37°C and directly kept on wet ice until further use.

### ovalbumin

Ovalbumin is incorporated as a candidate ingredient of immunogenic compositions comprising crossbeta structure. The ovalbumin is either serving as the antigen itself, to which an immune response should be directed, or ovalbumin is used as the crossbeta adjuvant part in immunogenic compositions, comprising a target antigen with a different amino-acid sequence. For this latter use, ovalbumin comprising crossbeta is combined with the target antigen, to which an immune response is desired. Crossbeta adjuvated ovalbumin is for example covalently coupled to the antigen of choice, using coupling techniques known to a person skilled in the art. When ovalbumin is the target antigen itself, non-treated ovalbumin and crossbeta-adjuvated ovalbumin are used in a similar way, in immunogenic composition preparations.

Lyophilized ovalbumin, or chicken egg-white albumin (OVA, Sigma, A5503 or A7641) is dissolved as follows. OVA is gently dissolved at indicated concentration in phosphate buffered saline (PBS; 140 mM sodium chloride, 2.7 mM potassium chloride, 10 mM disodium hydrogen phosphate, 1.8 mM potassium dihydrogen phosphate, pH 7.3; local pharmacy), avoiding any foam formation, stirring, vortexing or the like. OVA is dissolved by gently swirling, 10 minutes rolling on a roller device, 10 minutes warming in a 37°C-water bath, followed by 10 minutes rolling on a roller device. Aliquots in Eppendorf tubes are frozen at -80°C. Before use, OVA solution is either prepared freshly, or thawed from -80°C to 0°C, or after thawing kept at 37°C for 30 minutes. Furthermore, an OVA solution is applied to an endotoxin affinity matrix for removal of endotoxins present in the OVA preparation. Before and after applying OVA to the matrix, endotoxin levels are determined using an Endosafe apparatus (Charles River), and/or using a chromogenic assay for determining endotoxin levels (Cambrex), both using Limulus Amoebocyte Lysate (LAL). Misfolded OVA, termed *dOVA,* is prepared as indicated below (see Section "Protocols for introducing crossbeta in proteins").

### Hemagglutinin 5 protein of H5N1 virus strain A/Hong kong/156/97

Hemagglutinin 5 protein (H5) of H5N1 virus strain A/Hong kong/156/97 (A/HK/156/97) is expressed in 293 cells with a C-terminal FLAG tag and His tag, and purified using Ni²⁺-based affinity chromatography as described in patent application WO/2007/008070. In addition, the recombinantly produced H5-FLAG-His construct is purified using affinity chromatography with the anti-FLAG antibody M2 immobilized on a matrix (Sigma, A2220), according to the manufacturer's recommendations and using FLAG peptide (Sigma, F3290) for elution of H5-FLAG-His from the matrix. Protein solutions are stored at -80°C for a long term and after micro filtration at 4°C, for a short term. In this example, upon purification using anti-FLAG antibody based affinity chromatography, two batches of H5 were obtained. One batch of H5-FLAG-His is termed non-treated H5, batch 2 ('nH5-2', concentration 30 µg/ml). A second batch of H5-FLAG-His was subsequently subjected to size-exclusion chromatography (SEC) using a HiLoad 26/60 Superdex 200 column on an Äkta Explorer (GE Healthcare; used at the ABC-protein expression facilities of the University of Utrecht, Dr R. Romijn & Dr. W. Hemrika). For this purpose, H5-FLAG-His solution in PBS is concentrated on Macrosep Centrifugal Devices 10K Omega (Pall Life Sciences) or CENTRIPREP Centrifugal Filter Devices YM-300 (Amicon). Running buffer was PBS. The H5 batch after the SEC run, termed non-treated H5, batch 1 ('nH5-1'), was stored at 4°C after micro filtration (concentration 400 µg/ml, as determined with the BCA method). This batch nH5-1 is used for misfolding procedures described below.

### H5 of H5N1 strain A/Vietnam/1203/04

H5 of H5N1 strain A/Vietnam/1203/04 (A/VN/1203/04) is purchased from Protein Sciences, and consists mainly of HA2, with relatively lower amounts of HA1 and HA0. Purity is 90%, as determined with densitometry, according to the manufacturer's information. Buffer and excipients are 10 mM sodium phosphate, 150 mM NaCl, 0.005% Tween80, pH 7.2. The H5 concentration is 922 µg/ml (lot 45-05034-2) or 83 pg/ml (lot 45-05034RA-2). This non-treated H5 is termed 'nH5' and stored at 4°C or at -80°C.

### Other antigens

The proteins described above are used for preparation of immunogenic compositions. However, the disclosed technologies are by no means restricted to the generation of immunogenic compositions comprising OVA, FVIII, H5 of A/VN/1203/04 or A/HK/156/97, or E2. Examples that further disclose the described technologies and their applications, are also generated using other and/or additional peptides, polypeptides, proteins, glycoproteins, protein-DNA complexes, protein-membrane complexes and/or lipoproteins as a basis for immunogenic compositions. These peptides, polypeptides, proteins, glycoproteins, protein-DNA complexes, protein-membrane complexes and/or lipoproteins are the antigen component, the crossbeta-adjuvated component or both the antigen component and the crossbeta-adjuvated component of immunogenic compositions. The peptides, polypeptides, proteins, glycoproteins, protein-DNA complexes, protein-membrane complexes and/or lipoproteins are originating from amino-acid sequences unrelated to pathogens and/or diseases, when used as the crossbeta-adjuvated ingredient of an immunogenic composition, or are originating from amino-acid sequences that are related to and/or involved in and/or are part of pathogens, tumors, cardiovascular diseases, atherosclerosis, amyloidosis, autoimmune diseases, graft-versus-host rejection and/or transplant rejection, when they are part of the target antigen and/or are the crossbeta-adjuvated ingredient of an immunogenic composition. In fact, the disclosed technologies are applicable to any amino-acid sequence, either of the antigen, or of the crossbeta-adjuvant.

Non-limiting examples of peptides, polypeptides, proteins, glycoproteins, protein-DNA complexes, protein-membrane complexes and/or lipoproteins that are used as antigen and/or as crossbeta-adjuvant are for example virus surface proteins, bacterial surface proteins, pathogen surface exposed proteins, gp 120 of HIV, proteins of human papilloma virus, any of the neuramidase proteins or hemagglutinin proteins or any of the other proteins of any influenza strain, surface proteins of blue tongue virus, proteins of foot- and mouth disease virus, bacterial membrane proteins, like for example PorA of Neisseria meningitides, oxidized low density lipoprotein, tumor antigens, tumor specific antigens, amyloid-beta, antigens related to rheumatoid arthritis, B-cell surface proteins CD19, CD20, CD21, CD22, proteins suitable for serving as target for immunocastration, proteins of hepatitis C virus (HCV), proteins of respiratory syncytial virus (RSV), proteins specific for non small cell lung carcinoma, malaria antigens, proteins of hepatitis B virus.

### Protocols and procedures for misfolding proteins and introducing crossbeta in proteins

Peptides, polypeptides, proteins, glycoproteins, protein-DNA complexes, protein-membrane complexes and/or lipoproteins, in summary referred to as 'protein' throughout this section, are misfolded with the occurrence of crossbeta structure after subjecting them to various crossbeta-inducing procedures. Below, a summary is given of a non-limiting series of those procedures, which are preferably applied to the proteins used in immunogenic compositions.

Misfolding of proteins with the occurrence of crossbeta is induced using selected combinations of several parameters. The following parameters settings are applied for proteins:
a. protein concentrations ranging from 10 µg/ml to 30 mg/ml, and preferably between 25 µg/ml and 10 mg/ml,
b. pH between 0 and 14, and preferably at pH 1.5-2.5 and/or pH 6.5-7.5 and/or 11.5-12.5 and or at the iso-electric point (IEP) of a protein, and for example induced with HCl or NaOH, for example using 2-5 M stock solutions.
c. NaCl concentrations between 0 and 5000 mM, and preferably 125-175 mM
d. buffer selected from PBS, HEPES-buffered saline (20 mM HEPES, 137 mM NaCl, 4 mM KCl, pH 7,4), or no buffer (H₂O),
e. a reducing agent like dithiothreitol (DTT) or β-mercaptoethanol is incorporated in the reaction mixture, and
f. temperature gradients and temperature end-points for an indicated time frame, that are applied for selected time frames of 10 seconds up to 24 h, and with selected ranges between 0 and 120°C, and preferably between 4 and 95°C, with preferably steps of 0.1-5°C/minute for gradients.

Furthermore, protein misfolding is induced for example by, but not limited to, post-translational modifications like for example glycation, using for example carbohydrates, oxidation, using for example CuSO₄, citrullination, using for example using peptidylarginine deiminases, acetylation, sulfatation, (partial) de-sulfatation, (partial) de-glycosylation, enzymatic cleavage, polymerization, exposure to chaotropic agents like urea (for example 0.1-8 M) or guanidinium-HCl (for example 0.1-7 M).

Misfolding of proteins with appearance of crossbeta is also achieved upon subjecting proteins to exposure to adjuvants currently in use or under investigation for future use in immunogenic compositions. Proteins are exposed to adjuvants only, or the exposure to adjuvants is part of a multi-parameter misfolding procedure, designed based on the aforementioned parameters and conditions. Non-limiting examples of adjuvants that are implemented in protocols for preparation of immunogenic compositions comprising crossbeta are alum (aluminium-hydroxide and/or aluminium-phosphate), MF59, QS21, ISCOM matrix, ISCOM, saponin, QS27, CpG-ODN, flagellin, virus like particles, IMO, ISS, lipopolysaccharides, lipid A and lipid A derivatives, complete Freund's adjuvant, incomplete Freund's adjuvant, calcium-phosphate, Specol.

A typical method for induction of crossbeta conformation in a protein is designed as follows in a matrix format, from which preferably subsets of parameter settings are selected.
i. protein concentration is 40/200/1000 µg/ml
ii. pH is 2, 7, 12 and at the IEP of the protein
iii. DTT concentration is 0 or 200 mM
iv. NaCl concentration is 0 or 150 mM
v. urea concentration is 0/2/8 M
vi. buffer is PBS or HBS (with adjusted NaCl concentration and/or pH, when indicated)
vii. temperature gradient is
   a. constantly at 4°C/22°C-37°C/65°C for an indicated time
   b. from room temperature to 65°C/85°C, for 1 to 5 cycles

Subsets of selected parameter settings are for example as follows.
A. 1 mg/ml protein in PBS, pH 7.3, 200 mM DTT, 150 mM NaCl, kept at 37°C for 60 minutes
B. 200 µg/ml protein in PBS, 150 mM NaCl, heated in a cyclic manner for three cycli from 25°C to 85°C, at 0.5°C/minute, with varying pH's.

### Misfolding of E2

E2 protein is misfolded accompanied by introduction of crossbeta, by applying various parameter ranges, selected from described parameters a-f (see above). For example, E2 concentration ranges from 50 µg/ml to 2 mg/ml; selected pH is 2, 7.0-7.4 and 12; selected NaCl concentration is 0-500 mM, for example 0/50/150/500 mM; selected buffer is PBS or HBS or no buffer (H₂O); selected temperature gradient is for example as described for OVA, below. For example, E2 at approximately 300 µg/ml in PBS, heated in PCR cups in a PTC-200 thermal cycler (MJ Research, Inc.): 25°C for 20 seconds and subsequently heated (0.1°C/second) from 25°C to 85°C followed by cooling to 4°C for 2 minutes. This cycle is for example repeated twice (total number of cycles is 3). For example, E2 is subsequently stored at -20°C.

For the examples described below, non-treated E2 (nE2) at approximately 280 µg/ml in PBS was incubated at 25°C for 20 seconds and was subsequently gradiently heated (0.1°C/second) from 25°C to 85°C followed by cooling at 4°C for 2 minutes. This cycle was repeated twice and then, the E2 solution, referred to as crossbeta E2 (cE2) was stored at -20°C.

Structural differences and differences in crossbeta content between nE2 and cE2 were assessed using ThT fluorescence measurement, tPA/Plg activation analysis and TEM imaging. See Figure 2. From these graphs and figures it is clearly seen that the content of crossbeta in cE2 is increased when compared to nE2; both ThT fluorescence and tPA/Plg activating potential are increased. On the TEM images it is seen that cE2 appears as clustered and relatively large multimers with various sizes, whereas also nE2 displays assemblies of protein, though with smaller size and not clustered. Further analysis of crossbeta content and appearance, and further analysis of multimeric size and multimeric size distribution is assessed by subjecting the E2 samples to various of the aforementioned analyses for crossbeta determination and molecular structure and size determinations. Furthermore, various additional appearances of cE2 variants are generated by subjecting nE2 and/or nE2-FLAG-His to selected misfolding procedures as depicted above. For example, nE2 is used at 0.1 and 1 mg/ml, at pH 2/7/12, with/without DTT, for cyclic heat-gradients running from 4 to 85°C, for 1 to 5 cycles, resulting in 60 variants of cE2. These variants are subjected to analysis of binding of antibodies, for selecting those cE2 variants that combine the ability to bind functional antibodies (see below) with the presence of potent immunogenic crossbeta conformation. In addition, nE2 is for example coupled to dOVA standard and/or a different variant of misfolded OVA with proven potent crossbeta-adjuvating properties (see the section on OVA misfolding and OVA immunizations).

### Misfolding of OVA

OVA is for example misfolded with introduction of crossbeta using the following misfolding procedures:
1. 10 mg/ml OVA in PBS, heating from 25 to 85°C, 5°C/minute
2. 1 mg/ml OVA in PBS, heating from 25 to 85°C, 5°C/minute
3. 0.1 mg/ml OVA in PBS, heating from 25 to 85°C, 5°C/minute
4. 10 mg/ml OVA in HBS, heating from 25 to 85°C, 5°C/minute
5. 1 mg/ml OVA in HBS, heating from 25 to 85°C, 5°C/minute
6. 0.1 mg/ml OVA in HBS, heating from 25 to 85°C, 5°C/minute
7. similar to the above six methods 1-6, now with a cooling step from 85 back to 25°C, and again heating to 85°C (repeated twice)
8. similar to the above six methods 1-6, now with a heating rate of 0.1°C/minute, and a cooling step from 85 back to 25°C (1-5 cycles)
9. addition of a final concentration of 1% SDS to 1 mg/ml OVA; incubation at room temperature for 30 minutes - 16 h
10. addition of urea to 0.1-10 mg/ml OVA, to a final concentration of 2-8 M. Incubation for preferably 1-16 h at preferably 4-65°C. OVA solution is dialyzed against preferably H₂O or PBS or HBS, before further use.
11. constantly heating of preferably 0.1-10 mg/ml OVA in preferably PBS or HBS or H₂O, for preferably 1-72 h at preferably 4-100°C. For example 0.1 and 1 mg/ml in PBS, for 20 h at 65°C.
12. constantly heating of preferably 0.1-10 mg/ml OVA in PBS, for 10 minutes at 100°C. For example 0.1 and 1 and 10 mg/ml.
13. addition of a final concentration of 0.5% SDS to 1 mg/ml OVA; incubation for preferably 1-16 h at preferably 4-37°C, for example 1 h at room temperature.
14. Oxidation: addition of CuSO₄ to a final concentration of 1 mM and incubation for 24 h at 37°C. The oxidized OVA is dialyzed before further use.
15. incubation of 300 µg/ml OVA with 4 mM ascorbic acid, 40 µM CuCl₂, for 3 h, in NaPi buffer pH 7.4. Oxidation is stopped by adding EDTA from a 100 mM stock, to 1 mM final concentration. The oxidized OVA is dialyzed before further use.
16. pH of an OVA solution at 600 µg/ml in HBS is lowered to pH 2 by adding a suitable amount of HCl from a 5 M stock. The solution is subsequently kept at 37°C for 30 minutes. Then, the pH is adjusted with NaOH to pH 7-7.4.
17. pH of an OVA solution at 600 µg/ml in HBS is raised to pH 12 by adding a suitable amount of NaOH solution from a 5 M stock. The solution is subsequently kept at 37°C for 30 minutes. Then, the pH is adjusted with HCl back to pH 7-7.4.
18. For comparison with methods 16 and 17, the same final amount of NaCl is added, which is finally added to the solutions described in 16 and 17 by adding HCl/NaOH or NaOH/HCl, to OVA solution, after incubation for 30 minutes at 37°C.

OVA was subjected to the following misfolding procedure for inducing crossbeta conformation. OVA was dissolved in PBS to a concentration of 1.0 mg/ml. The solution was put on a roller device for 10 minutes at room temperature (RT), than 10 minutes at 37°C in a water bath and subsequently again for 10 minutes on the roller device (RT). Then, 200 µl aliquots of OVA solution was heat-treated in a PTC-200 PCR machine (MJ Research) as follows: five cycles of heating from 30°C to 85°C at 5°C/minute; cooling back to 30°C. After five cycles misfolded OVA, termed dOVA, was cooled to 4°C and subsequently stored at -80°C. This preparation of dOVA is used as a standard reference, termed 'standard', with crossbeta content that results in a maximal signal (arbitrarily set to 100%) in indicated crossbeta detecting assays, at a given concentration.

Crossbeta analyses are performed with dOVA standard at a regular basis in our laboratories. For example in Figures 2, 6, 7 and 10, dOVA standard is analyzed for its capacity to enhance ThT fluorescence, Congo red fluorescence, tPA/Plg activation. Furthermore, dOVA standard appears as clusters or strings of aggregated molecules with various sizes on TEM images (Figure 3). Further crossbeta analyses and multimeric distribution analyses using described methods are applied to the dOVA standard preparation and to additionally produced misfolded OVA variants, as depicted above.

### Misfolding of H5 of H5N1 strain A/HK/156/97

The H5-FLAG-His batch nH5-1, obtained after anti-FLAG antibody affinity chromatography and size exclusion chromatography, was subjected to two misfolding procedures.
A. A batch of 2 mg of nH5-1 (400 µg/ml in PBS, filtered through a 0.22 µm filter) was misfolded as follows. Aliquots of 120 µl of nH5-1 in PCR strips were incubated at 25°C for 20 seconds and subsequently heated (0.1°C/second) from 25°C to 85°C, followed by cooling at 4°C for 2 minutes. This cycle was repeated twice. Then, the H5 sample was pooled and stored at 4°C, and referred to as 'cH5-A'.
B. A second batch of 2 mg of nH5-1 was subjected to the following misfolding procedure. DTT was added from a sterile 1 M stock in H₂O to a final concentration of 100 mM. The sample was mixed by vortexing, and incubated for 1 h at 37°C (stove). Subsequently, the H5 samples was dialyzed three times for ∼3 h against 3 1 PBS under sterile conditions, at 4°C. For dialysis, Slide-a-lyzers with a molecular weight cut-off of < 10 kDa (Pierce) were used. The volume of the H5 sample, referred to as 'cH5-B', after recovery was unchanged with respect to the starting volume.

For structure analyses and for formulation of vaccine candidate solution, before use the nH5-1 and nH5-2 were centrifuged for 10 minutes at 16,000*g at room temperature. cH5-A and cH5-B were used without the centrifugation step.

The nH5-1 and cH5-B samples were analyzed on an analytical SEC column (U-Express Proteins, Utrecht, The Netherlands). For this purpose, approximately 80 µl of the 400 µg/ml stocks was applied to a Superdex200 10/30 column, connected to an Äkta Explorer (GE Healthcare). Running buffer was PBS. Samples were centrifuged for 20 minutes at 13,000*g before loading onto the column. The samples were run at a flow rate of 0.2 ml/minute and elution of protein was recorded by measuring absorbance at 280 nm.

The nH5-1 and nH5-2 preparations appear on SDS-PA gel and Western blot as multimers ranging from monomer up till aggregates that do not enter the gel (Figure 4). Upon treatment with DTT, these multimers monomerize, indicative for the covalent coupling of nH5 molecules through disulfide bonds (See Figure 4B). The cH5-A preparation appears with a similar pattern on gel and blot compared to the non-treated variants (Figure 4). In contrast, the cH5-B variant appears predominantly as monomers on gel and blot, with also dimers and oligomers present, but to a far lesser extent than seen in nH5-1, nH5-2 and cH5-A (Figure 4). This observation is reflected in the elution patterns of nH5-1 and cH5-B from the SEC column, depicted in Figure 5. The nH5-1 elutes as one peak in the flow-through of the column, whereas cH5-B elutes predominantly as a peak in the flow-through with a small peak at approximately the H5 monomer size. In conclusion, it appears that cH5-B comprises predominantly multimers that are more readily separated into smaller multimers and monomers, when compared to nH5-1, nH5-2 and cH5-A. Ultracentrifugation for 1 h at 100,000*g, which is used as a method to separate soluble oligomers of proteins from multimers that are precipitated in the pellet fraction, was applied to nH5-1, cH5-A and cH5-B (Figure 6). It appears that when nH5-1 is subjected to the g-forces, no molecules that contribute to the ThT fluorescence are pelleted, indicative for the presence of soluble oligomers comprising crossbeta, and the absence of insoluble aggregates with crossbeta. In contrast, by applying 100,000*g for 1 h on cH5-A and cH5-B, a fraction of the ThT fluorescence enhancement is lost, indicative for the removal of insoluble multimers with crossbeta from the solution. The remaining fraction of both H5 variants apparently comprises soluble multimers with crossbeta conformation. TEM images of nH5-1, nH5-2 and cH5-A, as depicted in Figure 6, show that all three H5 variants comprise multimers to a certain extent. The nH5-2 concentration is about 13-fold lower than the nH5-1 and cH5-A concentration, reflected in the lower density of multimers. When comparing nH5-1 and cH5-A, it is observed that cH5-A comprises less multimers but a higher number of larger multimers. These analyses of multimer size and size distribution are extended using more of the aforementioned techniques, and by incorporating more appearances of H5 after subjecting H5 solutions to various alternative misfolding procedures.

The nH5-1 and nH5-2 preparations comprise a considerable amount of crossbeta conformation, as depicted in Figure 7, showing ThT fluorescence enhancement, Congo red fluorescence enhancement and the ability to increase tPA/Plg activity for both non-treated H5 variants. When comparing cH5-A with cH5-B it is clear that cH5-A displays higher signals in the three crossbeta detecting assays. When comparing the patterns of the signals obtained in the three assays with the four H5 variants, it is seen that all four variants display a unique combination of signals, indicating that four different appearances and/or contents of crossbeta are present. H5 variants are subjected to further crossbeta analyses in order to obtain more insight in the different appearances of crossbeta upon subjecting H5 to varying misfolding conditions.

### Misfolding of H5 of H5N1 strain A/VN/1203/04

H5 of H5N1 strain A/VN/1203/04, as obtained from Protein Sciences, was subjected to four misfolding procedures, as indicated below.

### 1. nH5

For comparison, NaCl from a 5 M stock was added to non-treated H5 stock (922 µg/ml, 4°C, 150 mM NaCl), to a final concentration of 171 mM NaCl, and subsequently aliquoted in Eppendorf cups and stored at -20°C. Endotoxin level: <0.05 EU / 10 µg/ml solution nH5 (determined using an Endosafe pts apparatus (Charles River). The solution was clear and colorless. For structure analyses and for formulation of vaccine candidate solution, before use the nH5 was centrifuged for 10 minutes at 16,000*g at room temperature.

### 2. cH5-1

Aliquots of nH5 in PCR strips (Roche) were incubated at 25°C for 20 seconds and subsequently gradiently heated (0.1°C/second) from 25°C to 85°C followed by cooling back to 4°C, and kept at 4°C for 2 minutes. This heat cycle was repeated twice. Then, aliquots in Eppendorf 500 µL cups were stored at -20°C. Code: 'cH5-1'. The preparation cH5-1 was slightly turbid with some visible precipitates after heat treatment.

### 3. cH5-2

The pH of the nH5 stock kept at 4°C, was lowered to pH 2 by adding HCl from a 15% v/v stock. Then, aliquots of 100 µL/cup in PCR strips were heated in a PTC-200 thermal cycler, as follows. The samples were incubated at 25°C for 20 seconds and subsequently gradiently heated (0.1°C/second) from 25°C to 85°C followed by cooling back to 4°C, and kept at 4°C for 2 minutes. This heat cycle was repeated twice. Subsequently, the pH was adjusted to pH 7 by adding a volume NaOH solution from a 5 M stock. Then, aliquots in Eppendorf 500 µL cups were stored at -20°C. Code: 'cH5-2'. The solution was clear and colorless.

### 4. cH5-3

The pH of nH5 kept at 4°C, was elevated to pH 12 by adding a volume NaOH solution from a 5 M stock. Then, aliquots of 100 µL/cup in PCR strips were treated as follows in a PTC-200 thermal cycler. The samples were incubated at 25°C for 20 seconds and subsequently gradiently heated (0.1°C/second) from 25°C to 85°C followed by cooling back to 4°C, and kept at 4°C for 2 minutes. This heat cycle was repeated twice. Subsequently the pH was adjusted to pH 7 by adding a volume HCl solution from a 5 M stock. Then, aliquots in Eppendorf 500 µL cups were stored at -20°C. Code: 'cH5-3'. The solution was clear and colorless.

### 5. cH5-4

D-Glucose-6-phosphate disodium salt hydrate (g6p, Sigma; G7250) was added from a 2 M stock in PBS to nH5 to a final concentration of 100 mM g6p (20-fold dilution). Then it was incubated for 67 h at 80°C. The solution was intensively dialyzed against PBS, aliquoted in Eppendorf 500 µL cups, and stored at -20°C. The solution was light brown with white precipitates, visible by eye.

For structure analyses and for formulation of vaccine candidate solution, before use the nH5 was centrifuged for 10 minutes at 16,000*g at room temperature. cH5-1 to 4 were used without the centrifugation step.

The nH5 protein, as purchased from Protein Sciences, appears predominantly as the approximately 25 kDa HA2 fragment, with a smaller content of HA0 (full-length H5) and HA1 (molecular weight approximately 50 kDa) on reducing and non-reducing SDS-PA gels, stained with Coomassie (Figure 8).

The nH5 appears on a TEM image as amorphous multimers which are relatively small in size and which tend to aggregate into clusters, as seen in the supernatant after 10 minutes centrifugation at 16,000*g (Figure 9). In contrast, the four misfolded forms of H5, cH5-1 to 4, all appear as larger aggregates. The aggregates observed for cH5-1 and cH5-2 are similar in size and larger than the aggregates seen for cH5-3 and 4. Aggregates in cH5-2 seem to be more amorphous than the aggregates seen in cH5-2.

ThT fluorescence is enhanced with cH5-1 to 3, when compared to nH5 (Figure 10A). The non-treated nH5 still displays a significant ThT fluorescent signal. The signal is decreased for cH5-4, when compared to nH5. A similar pattern is seen for Congo red fluorescence (Figure 10B). The relative tPA/Plg activation potency of nH5 and cH5-1 to 4 displays a different pattern. cH5-2 and 3 enhance tPA/Plg activation to a somewhat larger extent than nH5, whereas cH5-1 and cH5-4 are less potent activators of tPA/Plg when compared to nH5 (Figure 10C). The five H5 forms are subjected to extended crossbeta analyses and extended multimer size and distribution analyses, in order to obtain more detailed information about the structural appearances.

All of the aforementioned antigens are preferably subjected to the described Disappearing Epitope Scanning approach for obtaining an immunogenic composition that comprises crossbeta and T-cell epitope motifs.

## Claims

1. A method for producing an immunogenic composition comprising at least one peptide, polypeptide, protein, glycoprotein, protein-DNA complex, protein-membrane complex and/or lipoprotein, the method comprising:
- determining whether a peptide, polypeptide, protein, glycoprotein, protein-DNA complex, protein-membrane complex and/or lipoprotein comprises a T-cell epitope motif;
- selecting a peptide, polypeptide, protein, glycoprotein, protein-DNA complex, protein-membrane complex and/or lipoprotein comprising a T-cell epitope motif;
- providing a composition comprising said selected peptide, polypeptide, protein, glycoprotein, protein-DNA complex, protein-membrane complex and/or lipoprotein; and
- providing said composition with at least one crossbeta structure.

2. A method for producing an immunogenic composition which is capable of activating a T-cell and/or a T-cell response, the composition comprising at least one peptide, polypeptide, protein, glycoprotein, protein-DNA complex, protein-membrane complex and/or lipoprotein comprising a T-cell epitope and/or a T-cell epitope motif, the method comprising providing said composition with at least one crossbeta structure and determining:
- whether the degree of multimerization of said peptide, polypeptide, protein, glycoprotein, protein-DNA complex, protein-membrane complex and/or lipoprotein in said composition allows recognition, binding, excision, processing and/or presentation of a T-cell epitope of said peptide, polypeptide, protein, glycoprotein, protein-DNA complex, protein-membrane complex and/or lipoprotein by an animal's immune system;
- whether between 4-75% of the peptide, polypeptide, protein, glycoprotein, protein-DNA complex, protein-membrane complex and/or lipoprotein content of said composition is in a conformation comprising crossbeta structures;
- whether said at least one crossbeta structure comprises a property allowing recognition, binding, excision, processing and/or presentation of a T-cell epitope of said peptide, polypeptide, protein, glycoprotein and/or lipoprotein by an animal's immune system; and/or
- whether a compound capable of specifically binding, recognizing, excising, processing and/or presenting a T-cell epitope of said peptide, polypeptide, protein, glycoprotein, protein-DNA complex, protein-membrane complex and/or lipoprotein is capable of specifically binding, recognizing, excising, processing and/or presenting said T-cell epitope.

3. A method according to any one of claims 1-2, comprising determining whether an MHC antigen processing pathway is capable of binding, recognizing, excising, processing and/or presenting a T-cell epitope of said peptide, polypeptide, protein, glycoprotein, protein-DNA complex, protein-membrane complex and/or lipoprotein.

4. A method according to any one of claims 1-3, comprising determining whether said crossbeta structure is capable of specifically binding a crossbeta structure binding compound, preferably tPA, BiP, factor XII, fibronectin, hepatocyte growth factor activator, at least one finger domain of tPA, at least one finger domain of factor XII, at least one finger domain of fibronectin, at least one finger domain of hepatocyte growth factor activator, Thioflavin T, Thioflavin S, Congo Red, CD14, a multiligand receptor such as RAGE or CD36 or CD40 or LOX-1 or TLR2 or TLR4, a crossbeta-specific antibody, preferably crossbeta-specific IgG and/or crossbeta-specific IgM, IgIV, an enriched fraction of IgIV capable of specifically binding a crossbeta structure, Low density lipoprotein Related Protein (LRP), LRP Cluster II, LRP Cluster IV, Scavenger Receptor B-I (SR-BI), SR-A, chrysamine G, a chaperone, a heat shock protein, HSP70, HSP60, HSP90, gp95, calreticulin, a chaperonin, a chaperokine and/or a stress protein.

5. A method according to any one of claims 1-4, further comprising selecting an immunogenic composition wherein the degree of multimerization of said peptide, polypeptide, protein, glycoprotein, protein-DNA complex, protein-membrane complex and/or lipoprotein allows recognition, binding, excision, processing and/or presentation of a T-cell epitope of said peptide, polypeptide, protein, glycoprotein, protein-DNA complex, protein-membrane complex and/or lipoprotein by an animal's immune system.

6. A method according to any one of claims 1-5, further comprising selecting an immunogenic composition wherein between 4-75% of the peptide, polypeptide, protein, glycoprotein, protein-DNA complex, protein-membrane complex and/or lipoprotein content of said composition is in a conformation comprising crossbeta structures.

7. A method according to any one of claims 1-6, further comprising selecting an immunogenic composition which comprises a crossbeta structure which is capable of specifically binding a crossbeta structure binding compound, preferably tPA, BiP, factor XII, fibronectin, hepatocyte growth factor activator, at least one finger domain of tPA, at least one finger domain of factor XII, at least one finger domain of fibronectin, at least one finger domain of hepatocyte growth factor activator, Thioflavin T, Thioflavin S, Congo Red, CD 14, a multiligand receptor such as RAGE or CD36 or CD40 or LOX-1 or TLR2 or TLR4, a crossbeta-specific antibody, preferably crossbeta-specific IgG and/or crossbeta-specific IgM, IgIV, an enriched fraction of IgIV capable of specifically binding a crossbeta structure, Low density lipoprotein Related Protein (LRP), LRP Cluster II, LRP Cluster IV, Scavenger Receptor B-I (SR-BI), SR-A, chrysamine G, a chaperone, a heat shock protein, HSP70, HSP60, HSP90, gp95, calreticulin, a chaperonin, a chaperokine and/or a stress protein.

8. A method according to any one of claims 1-7, further comprising selecting an immunogenic composition whereby a compound capable of binding, recognizing, excising, processing and/or presenting a T-cell epitope, preferably an MHC antigen processing pathway, is capable of binding, recognizing, excising, processing and/or presenting a T-cell epitope of said peptide, polypeptide, protein, glycoprotein, protein-DNA complex, protein-membrane complex and/or lipoprotein.

9. An *in vitro* method for selecting, from a plurality of immunogenic compositions comprising at least one crossbeta structure and at least one peptide and/or polypeptide and/or protein and/or glycoprotein and/or protein-DNA complex and/or protein-membrane complex and/or lipoprotein with a T-cell epitope or a T-cell epitope motif, one or more immunogenic compositions having a higher chance of being capable of eliciting a protective prophylactic cellular immune response and/or a therapeutic cellular immune response *in vivo,* as compared to the other immunogenic compositions of said plurality of immunogenic compositions, the method comprising:
selecting, from said plurality of immunogenic compositions, an immunogenic composition:
- wherein the degree of multimerization of said peptide, polypeptide, protein, glycoprotein, protein-DNA complex, protein-membrane complex and/or lipoprotein in said composition allows recognition, binding, excision, processing and/or presentation of a T-cell epitope of said peptide, polypeptide, protein, glycoprotein, protein-DNA complex, protein-membrane complex and/or lipoprotein by an animal's immune system;
- wherein between 4-75% of the peptide, polypeptide, protein, glycoprotein, protein-DNA complex, protein-membrane complex and/or lipoprotein content of said composition is in a conformation comprising crossbeta structures;
- which comprises a crossbeta structure which is capable of specifically binding a crossbeta structure binding compound, preferably tPA, BiP, factor XII, fibronectin, hepatocyte growth factor activator, at least one finger domain of tPA, at least one finger domain of factor XII, at least one finger domain of fibronectin, at least one finger domain of hepatocyte growth factor activator, Thioflavin T, Thioflavin S, Congo Red, CD14, a multiligand receptor such as RAGE or CD36 or CD40 or LOX-1 or TLR2 or TLR4, a crossbeta-specific antibody, preferably crossbeta-specific IgG and/or crossbeta-specific IgM, IgIV, an enriched fraction of IgIV capable of specifically binding a crossbeta structure, Low density lipoprotein Related Protein (LRP), LRP Cluster II, LRP Cluster IV, Scavenger Receptor B-I (SR-BI), SR-A, chrysamine G, a chaperone, a heat shock protein, HSP70, HSP60, HSP90, gp95, calreticulin, a chaperonin, a chaperokine and/or a stress protein; and/or
- whereby a compound capable of binding, recognizing, excising, processing and/or presenting a T-cell epitope, preferably an MHC antigen processing pathway, is capable of binding, recognizing, excising, processing and/or presenting a T-cell epitope of said peptide, polypeptide, protein, glycoprotein, protein-DNA complex, protein-membrane complex and/or lipoprotein.

10. A method according to any one of claims 1-9, wherein said crossbeta structure is induced in at least part of said peptide, polypeptide, protein, glycoprotein, protein-DNA complex, protein-membrane complex and/or lipoprotein.

11. A method according to any one of claims 1-10, wherein said at least one peptide, polypeptide, protein, glycoprotein, protein-DNA complex, protein-membrane complex and/or lipoprotein is subjected to a crossbeta inducing procedure, preferably a change of pH, salt concentration, temperature, buffer and/or chaotropic agent concentration.

12. A method according to any one of claims 1-11, wherein said at least one peptide, polypeptide, protein, glycoprotein, protein-DNA complex, protein-membrane complex and/or lipoprotein is coupled to a crossbeta comprising compound.

13. A method according to any one of claims 5-12, further comprising producing a vaccine comprising said selected immunogenic composition.

14. Use of an immunogenic composition produced and/or selected with a method according to any one of claims 1-12 as a vaccine or for the preparation of a vaccine.

15. An immunogenic composition comprising an immunogenic composition produced and/or selected with a method according to any one of claims 1-12.

16. An immunogenic composition according to claim 15, which is a vaccine.

17. Use of an immunogenic composition according to claim 15 or 16 for the preparation of a vaccine for the prophylaxis and/or treatment of a disorder caused by a pathogen, tumor, cardiovascular disease, atherosclerosis, amyloidosis, autoimmune disease, graft-versus-host rejection and/or transplant rejection.

18. A method for at least in part preventing and/or counteracting a disorder caused by a pathogen, tumor, cardiovascular disease, atherosclerosis, amyloidosis, autoimmune disease, graft-versus-host rejection and/or transplant rejection, comprising administering to a subject in need thereof a therapeutically amount of an immunogenic composition according to claim 15 or 16.

19. A method according to any one of claims 1-13, wherein said animal is a human individual.

20. A method according to any one of claims 1-13, wherein said T-cell epitope is a CTL epitope.

21. A method according to any one of claims 1-13, wherein said T-cell epitope is a T-helper cell epitope.

22. A method according to any one of claims 3-13, wherein said MHC antigen processing pathway is a MHC-I system.

23. A method according to any one of claims 3-13, wherein said MHC antigen processing pathway is a MHC-II system.

24. An immunogenic composition according to claim 15 or 16, further comprising a suitable carrier.

25. Use according to claim 14, wherein said vaccine comprises a suitable carrier.

26. A method according to any one of claims 1-13, comprising determining whether monomers and/or multimers of the peptide, polypeptide, protein, glycoprotein, protein-DNA complex, protein-membrane complex and/or lipoprotein in said immunogenic composition have dimensions in the range of 0.5 nm to 1000 µm, preferably in the range of 0.5 nm to 100 µm, more preferably in the range of 1 nm to 5 µm, and even more preferably in the range of 3 - 2000 nm.
